# EUROPEAN PATENT APPLICATION

(11) **EP 4 410 277 A1**
(43) Date of publication of application: **07.08.2024**
(21) Application number: 22876110.2
(22) Date of filing: 26.09.2022
(51) Int. Cl.: A61K 9/127, A61K 9/16, A61K 31/7088, A61K 47/20, A61K 47/22, A61K 47/24, A61K 47/28, A61K 47/34, A61K 47/42, A61K 48/00, A61P 35/00, A61P 43/00

(54) **LIPID NANOPARTICLES HAVING CELL DIRECTIVITY**

(30) Priority: 30.09.2021 JP 2021161235
(71) Applicant: NOF Corporation, Shibuya-ku Tokyo 150-6019 (JP); National University Corporation Chiba University, Chiba-shi, Chiba 263-8522 (JP)
(72) Inventor: TANGE, Kota, Kawasaki-shi, Kanagawa 210-0865 (JP); YOSHIOKA, Hiroki, Kawasaki-shi, Kanagawa 210-0865 (JP); NAKAI, Yuta, Kawasaki-shi, Kanagawa 210-0865 (JP); AKITA, Hidetaka, Chiba-shi, Chiba 260-8675 (JP); SAKURAI, Yu, Chiba-shi, Chiba 260-8675 (JP); TANAKA, Hiroki, Chiba-shi, Chiba 260-8675 (JP); IWASAKI, Koki, Chiba-shi, Chiba 260-8675 (JP)
(74) Representative: J A Kemp LLP
(86) International application number: PCT/JP2022/035639
(87) International publication number: WO 2023/054243

(57) **Abstract**

The present invention provides a lipid nanoparticle including an ionic lipid represented by the following formula (1) wherein the symbols are as defined in the specification and an activated PEG lipid-bonded ligand having cell directivity.

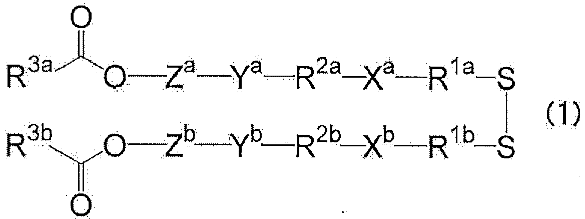

## Description

### [Technical Field]

The present invention relates to lipid nanoparticles having cell directivity, production methods thereof, and use thereof.

### [Background Art]

For practicalization of nucleic acid therapy, an effective and safe nucleic acid delivery carrier is demanded. While virus vectors are nucleic acid delivery carriers with good expression efficiency, they have practical problems from the aspect of safety. Therefore, the development of non-viral nucleic acid delivery carriers that can be used more safely is ongoing. Among them, lipid nanoparticles that are carriers using ionic lipids are non-viral nucleic acid delivery carriers most generally used at present.

Ionic lipids are largely constituted of amine moiety and lipid moiety. The amine moiety, which is protonated under acidic conditions, interacts electrostatically with nucleic acids, which are polyanions, to form lipid nanoparticles, which promotes uptake into cells and delivers nucleic acids into cells.

A known ionic lipid that is generally widely used is, for example, 1,2-dioleoyl-3-dimethylammonium propane (DODAP). It is known that by combining known ionic lipids with phospholipids, cholesterol, and PEG lipids, lipid nanoparticles can be formed and nucleic acids can be delivered into cells (Non Patent Literature 1).

Patent Literature 1 describes an ionic lipid having a structure in which compounds consisting of one or two amine moieties and one lipid moiety are connected by a biodegradable disulfide bond. This literature states that the ionic lipid can improve pharmacokinetics such as blood stability and tumor targeting, and that by changing the structure around the amine moiety, the pKa of a lipid membrane structure can be adjusted to a value advantageous for endosomal escape in cells, and further that it has the effect of dissociating nucleic acids from lipid membrane structures by utilizing the cleavage of disulfide bonds within cells. In fact, since it shows higher nucleic acid delivery efficiency compared to a known ionic lipid DODAP, it is clear that this ionic lipid can achieve improvement of improve the intracellular dynamics such as improvement of the delivery efficiency of nucleic acids into the cytoplasm and the like.

In Patent Literature 2, a lipid membrane structure is shown that has enhanced ability to fuse with endosomal membrane and has further improved efficiency of nucleic acid introduction into the cytoplasm, by using an ionic lipid having, in addition to a tertiary amine moiety and disulfide bond, an aromatic ring introduced near the lipid moiety.

Non Patent Literature 2 shows lipid nanoparticles modified with GALA peptide with the effect of increasing endosomal escape efficiency. This document shows that GALA-PEG lipids are synthesized in advance by bonding PEG lipids and GALA peptides, and then lipid nanoparticles are produced from GALA-PEG lipids and other components, whereby lipid nanoparticles modified with GALA peptides can be produced.

As described above, ionic lipids with improved intracellular dynamics have been developed by increasing endosomal escape efficiency and membrane fusion ability. On the other hand, in order for lipid nanoparticles made of ionic lipids to exhibit more practical effects as nucleic acid delivery carriers in vivo, directivity to target organs and cells is demanded.

Non Patent Literature 3 discloses lipid nanoparticles modified with RGD peptide as a ligand for imparting organ directivity. This document shows that RGD-PEG lipids are synthesized in advance by bonding PEG lipids and RGD peptides, then lipid nanoparticles made of other components are produced, and the obtained lipid nanoparticles and RGD-PEG lipids are incubated, whereby lipid nanoparticles modified with RGD peptides are produced, and intravenous injection thereof increases the efficiency of nucleic acid delivery to tumors.

As described above, there are known multiple lipid nanoparticles with improved intracellular dynamics and multiple lipid nanoparticles with organ directivity. However, practicalization of lipid nanoparticles with organ directivity requires further improvement in the efficiency of nucleic acid introduction into target cells. Furthermore, the method of ligand modification of lipid nanoparticles requires further improvement in terms of efficiency and simplicity.

### [Citation List]

### [Patent Literature]

[Patent Literature 1]
   US 2014/0335157 A1
[Patent Literature 2]
   WO 2019/188867 A1

### [Non Patent Literature]

[Non Patent Literature 1]
   Biomaterials 29 (24-25): 3477-96, 2008
[Non Patent Literature 2]
   Journal of Pharmaceutical Sciences 106 (2017) 2420-2427
[Non Patent Literature 3]
   Journal of Controlled Release 173 (2014) 110-118

### [Summary of Invention]

### [Technical Problem]

The present invention aims to provide lipid nanoparticles having cell directivity that could not be achieved by conventional techniques, to provide production methods of the lipid nanoparticles, to provide a nucleic acid-introducing agent containing the lipid nanoparticles, and to provide a method for introducing a nucleic acid into a specific cell by using the nucleic acid-introducing agent.

### [Solution to Problem]

The present inventors have conducted intensive studies in view of the above-mentioned problems and found that nucleic acids can be efficiently delivered to the target cells by imparting a ligand having cell directivity to lipid nanoparticles containing an ionic lipid that has a pKa suitable for endosomal escape and is specifically decomposed in a reducing environment within cells. They have further found, regarding the method of ligand modification to lipid nanoparticles, that a ligand can be efficiently bonded to the surface of the lipid nanoparticles by reacting the lipid nanoparticles and the lipid after producing lipid nanoparticles containing a PEG lipid having a reactive functional group.

The present inventors have confirmed that lipid nanoparticles modified with the ligand having cell directivity retain, after being efficiently taken up by target cells, the properties of ionic lipids that they have high membrane fusion ability in the endosomal environment, have high endosomal escape efficiency, and can efficiently deliver nucleic acids into the cytoplasm of target cells, and completed the present invention. The present invention based on this finding is as follows.

[1] A lipid nanoparticle comprising
   an ionic lipid represented by the formula (1): 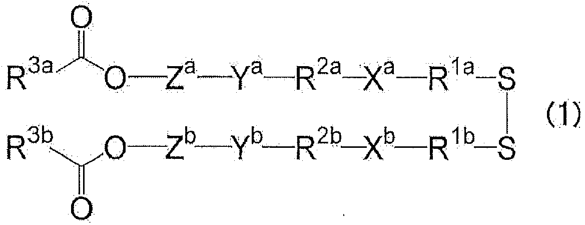 (in the formula (1),
   R^{1a} and R^{1b} are each independently an alkylene group having 1 to 6 carbon atoms,
   X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
   R^{2a} and R^{2b} are each independently an alkylene group or an oxydialkylene group each having not more than 8 carbon atoms,
   Y^{a} and Y^{b} are each independently an ester bond, an amide bond, a carbamate bond, an ether bond or a urea bond,
   Z^{a} and Z^{b} are each independently a divalent group derived from an aromatic compound having 3 to 16 carbon atoms and at least one aromatic ring, and optionally having a hetero atom, and
   R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms), and
   a ligand having cell directivity and bonded to an activated PEG lipid.
[2] The lipid nanoparticle of the aforementioned [1], wherein the activated PEG lipid is represented by the formula (2): 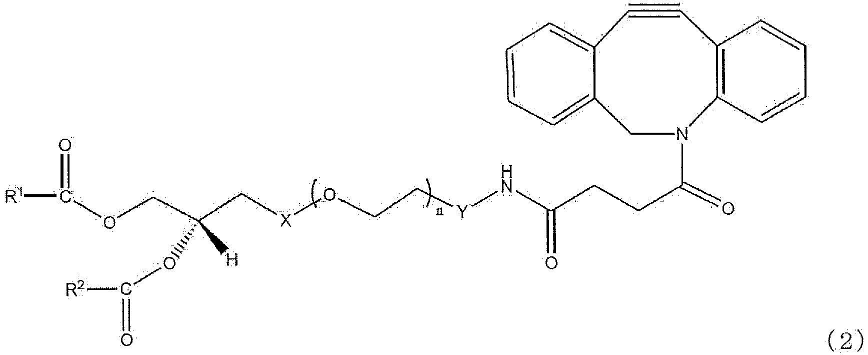 (in the formula (2),
   R¹ and R² are each independently a hydrocarbon chain having 14 to 18 carbon atoms,
   X is a group represented by the formula (3): 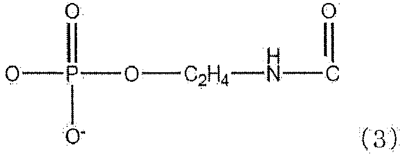 or a single bond,
   when X is a group represented by the formula (3), then Y is a single bond,
   when X is a single bond, then Y is an oxygen atom, and
   n is an integer that satisfies the molecular weight of (OCH₂CH₂)ₙ of 1000 to 5000.)
[3] The lipid nanoparticle of the aforementioned [2], wherein in the aforementioned formula (2), R¹ and R² are each a saturated hydrocarbon chain having 18 carbon atoms, X is a single bond, and Y is an oxygen atom.
[4] The lipid nanoparticle of any one of the aforementioned [1] to [3], wherein the ligand is a peptide or antibody that has specific affinity for a molecule expressed on the surface of the target cell.
[5] The lipid nanoparticle of the aforementioned [4], wherein the ligand is an anti-CD3 antibody.
[6] The lipid nanoparticle of the aforementioned [4], wherein the ligand is an anti-CD19 antibody.
[7] The lipid nanoparticle of any one of the aforementioned [1] to [5], wherein the ligand has directivity to T cells.
[8] The lipid nanoparticle of any one of the aforementioned [1] to [4] and [6], wherein the ligand has directivity to B cells.
[9] The lipid nanoparticle of any one of the aforementioned [1] to [8], wherein the ionic lipid represented by the formula (1) is O-Ph-P4C2 described in Table 1-1.
[10] A method for producing a lipid nanoparticle modified with a ligand having cell directivity, comprising
   producing a lipid nanoparticle from a lipid material comprising an ionic lipid represented by the formula (1): 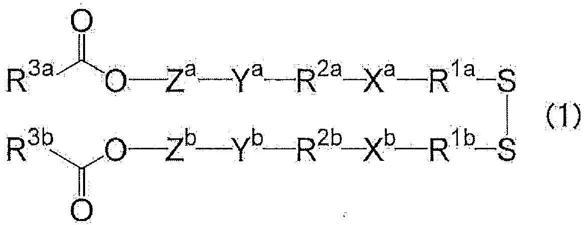 (in the formula (1),
   R^{1a} and R^{1b} are each independently an alkylene group having 1 to 6 carbon atoms,
   X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
   R^{2a} and R^{2b} are each independently an alkylene group or an oxydialkylene group each having not more than 8 carbon atoms,
   Y^{a} and Y^{b} are each independently an ester bond, an amide bond, a carbamate bond, an ether bond or a urea bond,
   Z^{a} and Z^{b} are each independently a divalent group derived from an aromatic compound having 3 to 16 carbon atoms and at least one aromatic ring, and optionally having a hetero atom, and
   R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms),
      and an activated PEG lipid, and
   binding the ligand to the obtained lipid nanoparticle.
[11] The method of the aforementioned [10], wherein the activated PEG lipid is represented by the formula (2): 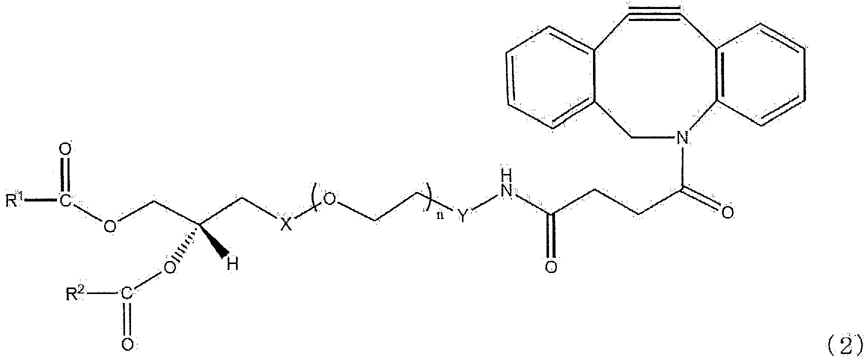 (in the formula (2),
   R¹ and R² are each independently a hydrocarbon chain having 14 to 18 carbon atoms,
   X is a group represented by the formula (3): 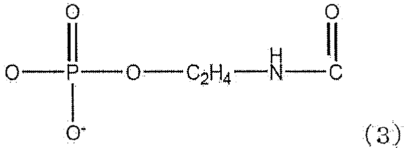 or a single bond,
      when X is the group represented by the formula (3), then Y is a single bond,
      when X is a single bond, then Y is an oxygen atom, and
      n is an integer that satisfies the molecular weight of (OCH₂CH₂)ₙ of 1000 to 5000).
[12] The method of the aforementioned [11], wherein in the aforementioned formula (2), R¹ and R² are each a saturated hydrocarbon chain having 18 carbon atoms, X is a single bond, and Y is an oxygen atom.
[13] The method of any one of the aforementioned [10] to [12], wherein the ionic lipid represented by the formula (1) is O-Ph-P4C2 described in Table 1-1.
[14] A nucleic acid-introducing agent comprising the lipid nanoparticles of any one of the aforementioned [1] to [9].
[15] A method for introducing a nucleic acid into a cell, comprising contacting the nucleic acid-introducing agent of the aforementioned [14] encapsulating the nucleic acid with the cell in vitro.
[16] The method of the aforementioned [15], wherein the cell is a T cell or a B cell.
[17] A method for introducing a nucleic acid into a cell, comprising administering the nucleic acid-introducing agent of the aforementioned [14] encapsulating the nucleic acid to a living body so that the agent is delivered to the target cell.
[18] The method of the aforementioned [17], wherein the target cell is a T cell or a B cell.

### [Advantageous Effects of Invention]

The present invention relates to lipid nanoparticles containing an ionic lipid having a tertiary amino group, a lipid moiety, and a disulfide bond as a biodegradable group, and a ligand having cell directivity. The lipid nanoparticles of the present invention can encapsulate any nucleic acid, and can also be used as a nucleic acid-introducing agent. In the lipid nanoparticles of the present invention, the ligand is recognized by the target cell and efficiently taken into the cell, and the disulfide bonds contained in the ionic lipid are cleaved within the cell, thereby promoting release of the inclusions (nucleic acid). Therefore, the nucleic acid-introducing agent of the present invention (lipid nanoparticles) can achieve high nucleic acid delivery efficiency into the cytoplasm of the target cell.

### [Brief Description of Drawings]

[Fig. 1]
   A diagram showing that LNP is taken up T cell-selectively in the spleen cells of a mouse to which an mRNA-encapsulating LNP modified with an anti-CD3 antibody fragment has been administered in vivo.
[Fig. 2]
   A diagram showing that LNP is efficiently taken up in the splenic T cells of a mouse to which an mRNA-encapsulating LNP modified with an anti-CD3 antibody fragment has been administered in vivo.
[Fig. 3]
   A diagram showing that introduced mRNA is efficiently expressed in the splenic T cells of a mouse to which an mRNA-encapsulating LNP modified with an anti-CD3 antibody fragment has been administered in vivo.
[Fig. 4]
   A diagram showing the uptake efficiency of mRNA-encapsulating and ligand-modified LNPs containing various activated PEG lipids into splenic T cells.
[Fig. 5]
   A diagram showing the uptake efficiency of mRNA-encapsulating and ligand-modified LNPs containing various activated PEG lipids into peripheral blood T cells.
[Fig. 6]
   A diagram showing the effect of activated PEG lipids (DSPE-PEG2k-DBCO and DSG-PEG2k-DBCO) on the expression efficiency of introduced mRNA in splenic T cells of a mouse to which an mRNA-encapsulating LNP modified with an anti-CD3 antibody fragment has been administered in vivo.
[Fig. 7]
   A diagram showing the effect of the concentration of activated PEG lipid (DSPE-PEG2k-DBCO) on the uptake of LNP into splenic B cells of a mouse to which an mRNA-encapsulating LNP modified with an anti-CD19 antibody fragment has been administered in vivo.
[Fig. 8]
   A diagram showing the effect of the concentration of activated PEG lipid (DSPE-PEG2k-DBCO) on the uptake of LNP into peripheral blood B cells of a mouse to which an mRNA-encapsulating LNP modified with an anti-CD19 antibody fragment has been administered in vivo.
[Fig. 9]
   A diagram showing the effect of the concentration of activated PEG lipid (DSG-PEG2k-DBCO) on the uptake of LNP into splenic B cells of a mouse to which an mRNA-encapsulating LNP modified with an anti-CD19 antibody fragment has been administered in vivo.
[Fig. 10]
   A diagram showing the effect of the concentration of activated PEG lipid (DSG-PEG2k-DBCO) on the uptake of an mRNA-encapsulating LNP modified with an anti-CD19 antibody fragment into peripheral blood B cells of a mouse to which the LNP has been administered in vivo.

### [Description of Embodiments]

While the embodiments of the present invention are explained in the following, the present invention is not limited thereto.

### Lipid nanoparticles

The present invention relates to lipid nanoparticles containing an ionic lipid represented by the formula (1) (i.e., ionic lipid having a tertiary amino group, a lipid moiety, and a disulfide bond as a biodegradable group), and further modified with a ligand having cell directivity. As used herein, being "modified with a ligand having cell directivity" means that the ligand is presented on the surface of the lipid nanoparticle so that the ligand will interact with molecules having affinity and expressed on the target cell and be delivered to the target cell. In the lipid nanoparticle of the present invention, the ligand is bound (preferably covalently bound) to an activated PEG lipid described below.

In the present specification, the "lipid nanoparticles" (Lipid Nano Particle, sometimes to be abbreviated as "LNP" in the present specification) means a particle having a membrane structure wherein the hydrophilic groups of amphiphilic lipids are arranged in the interface, facing the aqueous phase side. The "amphiphilic lipid" means a lipid having both a hydrophilic group and a hydrophobic group.

The particle size of the lipid nanoparticle of the present invention is not particularly limited as long as it is less than 1 µm and is preferably 10 nm to 500 nm, more preferably 30 nm to 300 nm. The particle size can be measured by using a particle size distribution measuring device such as Zetasizer Nano (Malvern) or the like. The particle size of the lipid nanoparticles can be appropriately adjusted by the method for producing the lipid nanoparticles. In the present specification, the "particle size" means an average particle size (number average) measured by a dynamic light scattering method.

Examples of the amphiphilic lipid include ionic lipid, phospholipid, PEG lipid, and the like. In the present specification, "PEG" means polyethylene glycol, "PEG lipid" means a lipid modified with PEG, and "Y modified with X " (e.g., X: PEG, Y: lipid) means Y bound by X. In the present specification, "PEG with a number average molecular weight of 2,000" is sometimes referred to as "PEG2k".

The lipid nanoparticles of the present invention may contain at least one selected from the group consisting of sterol, PEG lipid, and phospholipid as a constituent component in addition to the ionic lipid represented by the formula (1).

### Ionic lipid

The ionic lipid used in the present invention is an ionic lipid represented by the following formula (1) (sometimes to be abbreviated as "ionic lipid (1)" in the present specification). Only one kind of ionic lipid (1) may be used, or two or more kinds thereof may be used in combination.

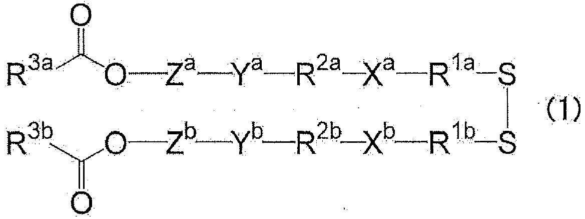

(in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom, and
R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms).

R^{1a} and R^{1b} are each independently an alkylene group having 1 to 6 carbon atoms, and may be linear or branched, preferably linear. The carbon number of the alkylene group is preferably 1 to 4, more preferably 1 to 2. Specific examples of the alkylene group having 1 to 6 carbon atoms include methylene group, ethylene group, trimethylene group, isopropylene group, tetramethylene group, isobutylene group, pentamethylene group, neopentylene group, and the like. Preferably, R^{1a} and R^{1b} are each independently a methylene group, an ethylene group, a trimethylene group, an isopropylene group, or a tetramethylene group, most preferably an ethylene group.

R^{1a} may be the same as or different from R^{1b}, and R^{1a} is preferably the same group as R^{1b}.

X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups, preferably each independently a cyclic alkylene tertiary amino group having 2 to 5 carbon atoms and 1 to 2 tertiary amino groups.

The alkyl group having 1 to 6 carbon atoms in the acyclic alkyl tertiary amino group having 1 to 6 carbon atoms and one tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like, preferably methyl group, ethyl group, propyl group, or isopropyl group, most preferably methyl group.

A preferred specific structure of the acyclic alkyl tertiary amino group having 1 to 6 carbon atoms and one tertiary amino group is represented by X¹.

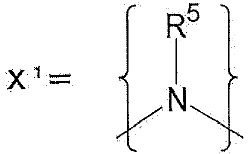

R⁵ in X¹ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like, preferably methyl group, ethyl group, propyl group, or isopropyl group, most preferably methyl group.

The carbon number of the cyclic alkylene tertiary amino group having 2 to 5 carbon atoms and 1 to 2 tertiary amino groups is preferably 4 to 5. The cyclic alkylene tertiary amino group having 2 to 5 carbon atoms and 1 to 2 tertiary amino groups is specifically an aziridylene group, an azetidylene group, a pyrrolidylene group, a piperidylene group, an imidazolidylene group, or a piperazylene group, preferably a pyrrolidylene group, a piperidylene group, or a piperazylene group, most preferably a piperidylene group.

A preferred specific structure of the cyclic alkylene tertiary amino group having 2 to 5 carbon atoms and one tertiary amino group is represented by X².

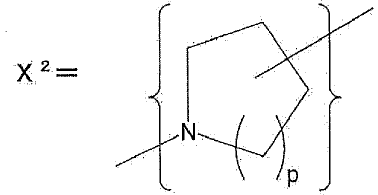

The p in X² is 1 or 2. When p is 1, X² is a pyrrolidylene group, and when p is 2, X² is a piperidylene group. Preferably, p is 2.

A preferred specific structure of the cyclic alkylene tertiary amino group having 2 to 5 carbon atoms and two tertiary amino groups is represented by X³.

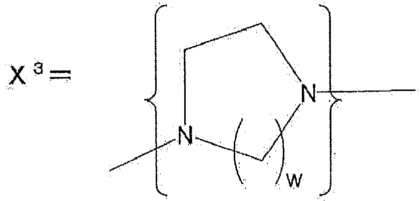

The w in X³ is 1 or 2. When w is 1, X³ is an imidazolidylene group, and when w is 2, X³ is a piperazylene group.

X^{a} may be the same as or different from X^{b}, and X^{a} is preferably the same group as X^{b}.

R^{2a} and R^{2b} are each independently an alkylene group or an oxydialkylene group each having not more than 8 carbon atoms, preferably each independently an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, trimethylene group, isopropylene group, tetramethylene group, isobutylene group, pentamethylene group, hexamethylene group, heptamethylene group, octamethylene group, and the like, preferably methylene group, ethylene group, trimethylene group, or tetramethylene group, most preferably ethylene group.

In the present specification, the "oxydialkylene group having not more than 8 carbon atoms" means alkylene groups via an ether bond (alkylene-O-alkylene, in other words, "alkyleneoxyalkylene group"), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group), and the like. Preferably, it is an oxydimethylene group, an oxydiethylene group, or an oxydi(tetramethylene) group, most preferably an oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 to 16 carbon atoms and at least one aromatic ring, and optionally having a hetero atom. The number of carbons contained in the aromatic compound is preferably 6 to 12, most preferably 6 to 7. The aromatic ring contained in the aromatic compound is preferably one.

As the kind of the aromatic ring contained in the aromatic compound having 3 to 16 carbon atoms, benzene ring, naphthalene ring, and anthracene ring can be mentioned for aromatic hydrocarbocycle, and imidazole ring, pyrazole ring, oxazole ring, isoxazole ring, thiazole ring, isothiazole ring, triazine ring, pyrrole ring, furan ring, thiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring, and the like can be mentioned for aromatic heterocycle. It is preferably a benzene ring, a naphthalene ring, or an anthracene ring, most preferably a benzene ring.

The aromatic ring may have a substituent. Examples of the substituent include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group, alkylureido group having 2 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like.

A preferred specific structure of Z^{a} and Z^{b} is Z¹. 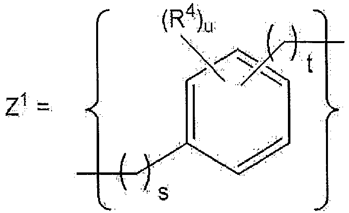 wherein s is an integer of 0 to 3, t is an integer of 0 to 3, u is an integer of 0 to 4, and R⁴ in the number of u are each independently a substituent.

The s in Z¹ is preferably an integer of 0 to 1, more preferably 0.

The t in Z¹ is preferably an integer of 0 to 2, more preferably 1.

The u in Z¹ is preferably an integer of 0 to 2, more preferably an integer of 0 to 1.

The R⁴ in Z¹ is a substituent of an aromatic ring (benzene ring) contained in the aromatic compound having 3 to 16 carbon atoms which does not inhibit the reaction in the synthesis process of an ionic lipid. Examples of the substituent include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group, alkylureido group having 2 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like. When R⁴ is present in plurality, each R⁴ may be the same or different.

Z^{a} may be the same as or different from Z^{b}, and Z^{a} is preferably the same group as Z^{b}.

R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms, preferably each independently a residue derived from a reaction product of a liposoluble vitamin having a hydroxyl group, and succinic anhydride or glutaric anhydride, or an aliphatic hydrocarbon group having 12 to 22 carbon atoms, most preferably each independently an aliphatic hydrocarbon group having 12 to 22 carbon atoms.

Examples of the liposoluble vitamin having a hydroxyl group include retinol, ergosterol, 7-dehydrocholesterol, calciferol, cholecalciferol, dihydroergocalciferol, dihydrotachysterol, tocopherol, tocotrienol, and the like. The liposoluble vitamin having a hydroxyl group is preferably tocopherol.

Examples of the sterol derivative having a hydroxyl group include cholesterol, cholestanol, stigmasterol, β-sitosterol, lanosterol, ergosterol, and the like, preferably cholesterol or cholestanol.

The aliphatic hydrocarbon group having 12 to 22 carbon atoms may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 to 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 13 to 19, most preferably 13 to 17. While the aliphatic hydrocarbon group includes an alkyl group, an alkenyl group, an alkynyl group, and the like, it is preferably an alkyl group or an alkenyl group. Specific examples of the aliphatic hydrocarbon group having 12 to 22 carbon atoms include dodecyl group, tridecyl group, tetradecyl group, pentadecyl group, hexadecyl group, heptadecyl group, octadecyl group, nonadecyl group, icosyl group, henicosyl group, docosyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, decadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, and the like. The aliphatic hydrocarbon group having 12 to 22 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In one embodiment of the present invention, the aliphatic hydrocarbon group having 12 to 22 carbon atoms for R^{3a} or R^{3b} is derived from fatty acid. In this case, the carbonyl carbon derived from fatty acid is contained in -CO-O- in the formula (1). A specific example of the aliphatic hydrocarbon group is a heptadecadienyl group when linoleic acid is used as the fatty acid, or a heptadecenyl group when oleic acid is used as the fatty acid.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferred examples of ionic lipid (1) include the following ionic lipids (1-1) to (1-3).

### [Ionic lipid (1-1)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-); and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 to 22 carbon atoms (e.g., heptadecenyl group, heptadecadienyl group, 1-hexylnonyl group).

### [Ionic lipid (1-2)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidylene group);
R^{2a} and R^{2b} are each independently an alkylene group having not more than 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-) ; and
R^{3a} and R^{3b} are each independently a residue derived from a reaction product of a liposoluble vitamin having a hydroxyl group (e.g., tocopherol), and succinic anhydride, or an aliphatic hydrocarbon group having 13 to 19 carbon atoms (e.g., heptadecenyl group, heptadecadienyl group, 1-hexylnonyl group).

### [Ionic lipid (1-3)]

Ionic lipid (1) wherein
R^{1a} and R^{1b} are each independently an alkylene group having 1 to 2 carbon atoms (e.g., methylene group, ethylene group);
X^{a} and X^{b} are each independently X¹: 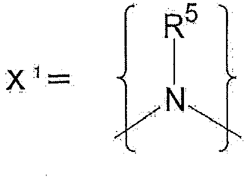 wherein R⁵ is an alkyl group having 1 to 3 carbon atoms (e.g., a methyl group), or X²: 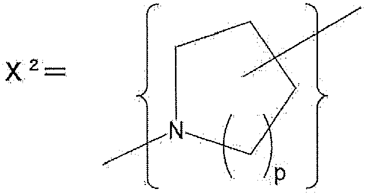 wherein p is 1 or 2;
R^{2a} and R^{2b} are each independently alkylene group having not more than 4 carbon atoms (e.g., methylene group, ethylene group, trimethylene group);
Y^{a} and Y^{b} are each independently an ester bond or an amide bond;
Z^{a} and Z^{b} are each independently Z¹: 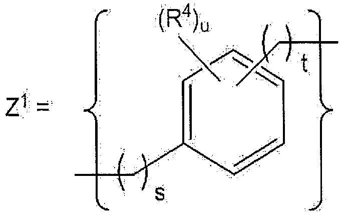 wherein s is an integer of 0 to 1, t is an integer of 0 to 2, u is an integer of 0 to 2 (preferably 0), and R⁴ in the number of u are each independently a substituent; and
R^{3a} and R^{3b} are each independently a residue derived from a reaction product of a liposoluble vitamin having a hydroxyl group (e.g., tocopherol) and succinic anhydride, or an aliphatic hydrocarbon group having 13 to 17 carbon atoms (e.g., heptadecenyl group, heptadecadienyl group, 1-hexylnonyl group).

Specific examples of ionic lipid (1) include the following O-Ph-P3C1, O-Ph-P4C1, O-Ph-P4C2, O-Bn-P4C2, E-Ph-P4C2, L-Ph-P4C2, HD-Ph-P4C2, O-Ph-amide-P4C2, and O-Ph-C3M. Among the specific examples of ionic lipid (1), O-Ph-P4C2 is preferred.

**[Table 1-1]**

| name of ionic lipid | structure |
|---|---|
| O-Ph-P3C1 | 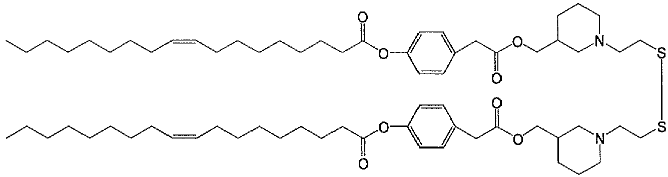 |
| O-Ph-P4C1 | 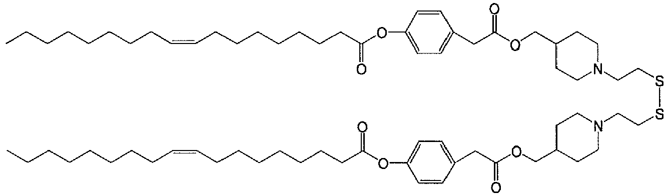 |
| O-Ph-P4C2 (or SS-OP) | 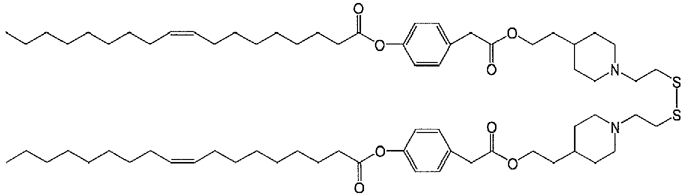 |
| O-Bn-P4C2 | 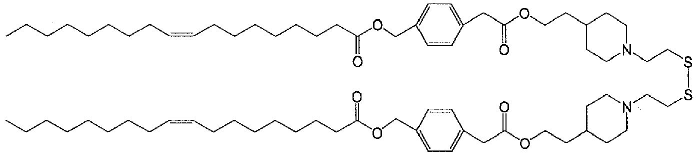 |
| E-Ph-P4C2 (or SS-EP) | 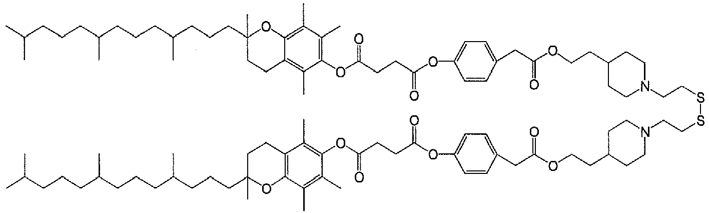 |

**[Table 1-2]**

| name of ionic lipid | structure |
|---|---|
| L-Ph-P4C2 | 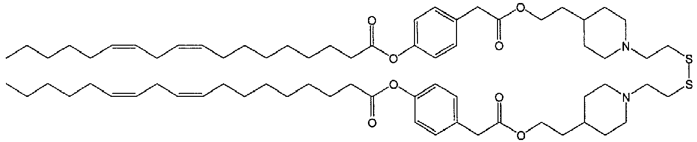 |
| HD-Ph-P4C2 | 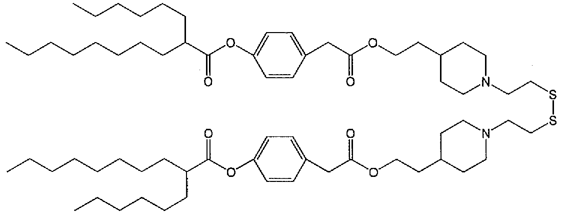 |
| O-Ph-amide-P4C2 | 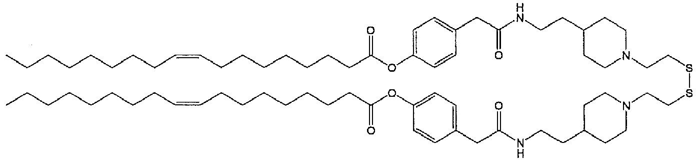 |
| O-Ph-C3M | 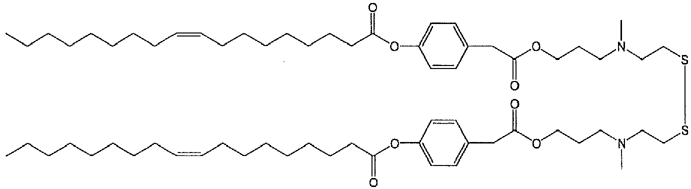 |

The amount of the ionic lipid (1) in the lipid nanoparticle of the present invention is preferably 10 to 90 mol%, more preferably 20 to 80 mol%, further preferably 30 to 70 mol%, with respect to the total amount of lipids in the lipid nanoparticle, from the aspects of efficiency of nucleic acid encapsulation, efficiency of intracellular release of nucleic acid, and stability of the lipid nanoparticles. As used herein, when, for example, lipid nanoparticles contain, in addition to ionic lipid (1), the below-mentioned phospholipid, sterol, and PEG lipid (including activated PEG lipid and non-activated PEG lipid) as constituent components, "the total amount of lipids in the lipid nanoparticle" means "the total amount of ionic lipid (1), the below-mentioned phospholipid, sterol, and PEG lipid". In the present specification, the "amount of B (mol%) with respect to A" means the "100 × amount of B (mol)/amount of A (mol)". For example, the "amount of ionic lipid (1) (mol%) with respect to the total amount of lipids" means the "100 × amount of ionic lipid (1) (mol)/total amount of lipid (mol)".

The ionic lipid (1) can be produced by a known method (e.g., the method described in WO 2019/188867 A1).

### Phospholipid

Phospholipid can be used as a constituent component of the lipid nanoparticles of the present invention. Only one kind of the phospholipid may be used, or two or more kinds thereof may be used in combination.

Examples of the phospholipid include 1,2-diacyl-sn-glycero-3-phosphocholine (PC), 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), 1,2-diacyl-sn-glycero-3-phosphatidic acid (PA), lyso forms of these, and the like.

Specific examples of 1,2-diacyl-sn-glycero-3-phosphocholine (PC) include 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), and 1-stearoyl-2-oleoyl-sn-glycero-3-phosphocholine (SOPC). It is also possible to use 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), and 1,2-diacyl-sn-glycero-3-phosphatidic acid (PA), in which the 3-position phosphocholine of those 1,2-diacyl-sn-glycero-3-phosphocholines is substituted with phosphoethanolamine, phosphoserine, phosphoglycerol, and phosphatidic acid, respectively.

Phospholipid is preferably PC and/or PE, more preferably at least one selected from the group consisting of DOPC, POPC, DOPE (1,2-dioleoyl-sn-glycero-3-phosphoethanolamine), and POPE 1-palmitoyl-2-oleoyl-sn-glycero-3-phosphoethanolamine) .

When phospholipid is used, the amount thereof in the lipid nanoparticle of the present invention is preferably 1 to 50 mol%, more preferably 1 to 30 mol%, further preferably 1 to 10 mol%, with respect to the total amount of lipids in the lipid nanoparticle, from the aspects of efficiency of nucleic acid encapsulation, efficiency of intracellular release of nucleic acid, and stability of the lipid nanoparticles.

### Sterol

Sterol can be used as the constituent component of the lipid nanoparticles of the present invention (particularly as component that adjusts the fluidity of lipid membranes). Only one kind of sterol may be used, or two or more kinds thereof may be used in combination.

Examples of the sterol include cholesterol, lanosterol, phytosterol, zymosterol, zymostenol, desmosterol, stigmastanol, dihydrolanosterol, and 7-dehydrocholesterol. The sterol is preferably at least one selected from the group consisting of cholesterol, lanosterol, and phytosterol, more preferably cholesterol.

When sterol is used, the amount thereof in the lipid nanoparticle of the present invention is preferably 1 to 70 mol%, more preferably 10 to 60 mol%, further preferably 30 to 50 mol%, with respect to the total amount of lipids in the lipid nanoparticle, from the aspects of efficiency of nucleic acid encapsulation, efficiency of intracellular release of nucleic acid, and stability of the lipid nanoparticles.

### PEG lipid

PEG lipid can be used, for example, to coat the surface of lipid nanoparticles with hydrophilic PEG in order to suppress the aggregation of lipid nanoparticles and in order to suppress the interaction of living organism components and the lipid nanoparticles when the particles are administered to the living organism. Only one kind of the PEG lipid may be used, or two or more kinds thereof may be used in combination.

The molecular weight of the PEG moiety of the PEG lipid used in the present invention is not particularly limited. The number average molecular weight (Mn) of the PEG moiety of the PEG lipid is, for example, 200 to 10,000. This number average molecular weight is a value analyzed by gel permeation chromatography (GPC), and is a value calculated based on a calibration curve created using polyethylene glycol of various known molecular weights (in the following, unless otherwise specified and no contradiction exists, references to "molecular weight" with respect to PEG mean number average molecular weight). The PEG moiety of the PEG lipid may be linear or branched.

Examples of the PEG lipid include PEG-bonded phospholipid (PEG-phospholipid), PEG-bonded ceramide (PEG-ceramide), PEG-bonded diacyl glycerol (PEG-diacyl glycerol), and PEG-bonded cholesterol (PEG-cholesterol). The PEG lipid is preferably a diacyl glycerol bonded with PEG having a number average molecular weight of 1,000 to 10,000, more preferably at least one selected from the group consisting of a dimyristoyl glycerol bonded with PEG having a number average molecular weight of 1,000 to 10,000 and a distearoyl glycerol bonded with PEG having a number average molecular weight of 1,000 to 10,000.

### Activated PEG lipid

The lipid nanoparticles of the present invention include activated PEG lipid. As used herein, the "activated PEG lipid" refers to a PEG lipid having a reactive group capable of binding to a ligand at the end of the PEG moiety. In order to distinguish from this activated PEG lipid, a PEG lipid that does not have a reactive group capable of binding to a ligand may be referred to as a "non-activated PEG lipid" in the present specification. Only one kind of activated PEG lipid and non-activated PEG lipid may be used, or two or more kinds thereof may be used in combination.

The amount of the activated PEG lipid in the lipid nanoparticle of the present invention is preferably 0.1 to 30 mol%, more preferably 0.5 to 20 mol%, further preferably 0.5 to 10 mol%, with respect to the total amount of lipids in the lipid nanoparticle, from the aspects of efficiency of nucleic acid encapsulation, efficiency of intracellular release of nucleic acid, stability of the lipid nanoparticles, and ligand modification efficiency.

When non-activated PEG lipid is used, the amount thereof in the lipid nanoparticle of the present invention is preferably 0.1 to 30 mol%, more preferably 0.5 to 20 mol%, further preferably 0.5 to 10 mol%, with respect to the total amount of lipidsin the lipid nanoparticle, from the aspects of efficiency of nucleic acid encapsulation, efficiency of intracellular release of nucleic acid, and stability of the lipid nanoparticles.

Examples of the activated PEG lipid include the aforementioned PEG lipids (e.g., PEG-phospholipid (preferably PEG-1,2-diacyl-sn-glycero-3-phosphoethanolamine (PE)), PEG-diacyl glycerol, PEG-cholesterol, etc.) with azide, maleimide, NHS (N-hydroxysuccinimide), DBCO (N-dibenzocyclooctyl), and BCN (bicyclo[6.1.0]nonyne) introduced into the PEG terminal.

In one preferred embodiment, the activated PEG lipid is a compound represented by the formula (2): 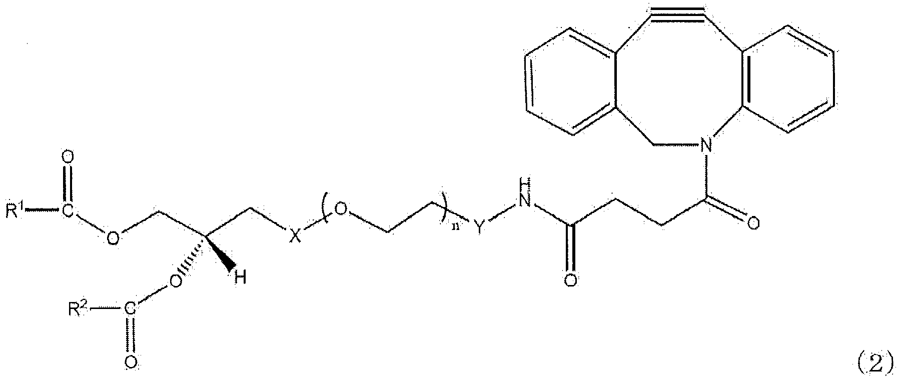 (in the formula (2),
R¹ and R² are each independently a hydrocarbon chain having 14 to 18 carbon atoms,
X is a group represented by the formula (3): 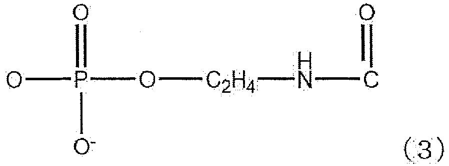
or a single bond,
when X is the group represented by the formula (3), then Y is a single bond,
when X is a single bond, then Y is an oxygen atom, and
n is an integer of one or two or more such that the molecular weight of (OCH₂CH₂)ₙ is 1000 to 5000) (hereinafter also to be referred to as "activated PEG lipid (2)").

Preferably, the activated PEG lipid is a compound of the formula (2), wherein R¹ and R² are each a saturated hydrocarbon chain having 18 carbon atoms, X is a single bond, and Y is an oxygen atom (DSG-PEG-DBCO), or a compound of the formula (2), wherein R¹ and R² are each a saturated hydrocarbon chain having 18 carbon atoms, X is a group represented by the formula (3), and Y is a single bond (DSPE-PEG-DBCO). It is more preferably DSG-PEG-DBCO.

The molecular weight of PEG is not particularly limited as long as it is within the above-mentioned range, and may be preferably 2000 (2k). Therefore, in a particularly preferred embodiment, the activated PEG lipid may be DSG-PEG2k-DBCO or DSPE-PEG2k-DBCO, more preferably DSG-PEG2k-DBCO. DSPE-PEG2k-DBCO is commercially available. Synthetic Example of DSG-PEG2k-DBCO is described below.

### Synthetic Example of DSG-PEG2k-DBCO

DSG-PEG2k-NHS (manufactured by NOF CORPORATION, product name SUNBRIGHT (registered trade mark) GS-020TS, 53 mg, 0.018 mmol) was dissolved in anhydrous dichloromethane (10 mL) under an argon atmosphere. To this solution was added N-(1R,8S,9s)-bicyclo[6.1.0]non-4-yn-9-ylmethyloxycarbonyl-1,8-diamino-3,6-dioxaoctane (7 mg, 0.020 mmol), and the mixture was stirred overnight. The obtained solution was partitioned twice with pure water (10 mL) and once with saturated brine. The solvent of the organic phase was evaporated to solidness, and the solid was dissolved in pure water, the reaction solution was transferred to a dialysis membrane with a cut-off molecular weight of 3.5 kDa, and dialyzed overnight against pure water. The dialysis solution was freeze-dried to evaporate the solvent to give a white solid (30 mg).

In another embodiment, the activated PEG lipid may be cholesterol (Chol)-PEG-BCN or dicholesterol (DiChol)-PEG-BCN. The molecular weight of PEG is not particularly limited as long as it is within the range of 1000 to 5000, and it is preferably 2000 (2k). Therefore, in a preferred embodiment, the activated PEG lipid may be Chol-PEG2k-BCN or DiChol-PEG2k-BCN. Synthetic Examples of these two compounds are described below.

### Synthetic Example of Chol-PEG2k-BCN

Cholesterol chloroformate (180 mg, 0.40 mmol) was dissolved in anhydrous dichloromethane (30 mL) under an argon atmosphere. To this solution were added PEG-diamine (number average molecular weight of PEG: 2,000) (500 mg, 0.25 mmol) and diazabicycloundecene (100 µL), and the mixture was stirred overnight. The obtained solution was partitioned once with 5 wt% sodium hydrogen carbonate aqueous solution (30 mL), twice with pure water (30 mL), and once with saturated brine (30 mL). The obtained solution was dried over sodium sulfate powder, and the organic layer was separated by flash chromatography (dichloromethane/methanol gradient). The solvent was evaporated from the separated solution to give a white-yellow solid (0.507 g).

The whole amount of the obtained solid was dissolved in anhydrous dichloromethane (10 mL) under an argon atmosphere, (1R,8S,9s)-bicyclo[6.1.0]non-4-yn-9-ylmethyl succinimidyl carbonate (70 mg), triethylamine (100 µL), 1-(3-dimethylaminopropyl)-3-ethylcarbodiimide hydrochloride (250 mg), N-hydroxysuccinic acid (100 mg), and dimethylaminopyridine (10 mg) were added, and the mixture was stirred overnight. The obtained solution was partitioned once with 5 wt% sodium hydrogen carbonate aqueous solution (30 mL), twice with pure water (30 mL), and once with saturated brine (30 mL). The obtained solution was dried over sodium sulfate powder, and the organic layer was separated by flash chromatography (hexane/ethyl acetate gradient). The solvent was evaporated from the separated solution to give Chol-PEG2k-BCN (0.596 g) as a white solid.

### Synthetic Example of DiChol-PEG2k-BCN

Cholesterol (1.08 g) was dissolved in anhydrous pyridine (20 mL) under an argon atmosphere. To this solution was added methanesulfonyl chloride (0.418 mg) under ice-cooling and the mixture was stirred overnight at room temperature. After evaporating the solvent, the residue was dissolved in ethyl acetate, and partitioned once with 5 wt% sodium hydrogen carbonate aqueous solution (30 mL), twice with pure water (30 mL), and once with saturated brine (30 mL). The obtained solution was dried over sodium sulfate powder and the organic layer was separated by flash chromatography (hexane/ethyl acetate gradient). The solvent was evaporated from the separated solution to give a white-yellow solid (0.432 g).

The whole amount of the obtained solid was dissolved in anhydrous tetrahydrofuran (30 mL) under an argon atmosphere, N-(tert-butoxycarbonyl)-serine (0.610 g) and potassium hydroxide (160 mg) were added, and the mixture was stirred overnight. The obtained solution was dried over sodium sulfate powder and separated by flash chromatography (hexane/ethyl acetate gradient). The solvent was evaporated from the separated solution to give a white solid (0.231 g).

The whole amount of the obtained solid was dissolved in dichloromethane (30 mL), trifluoroacetic acid (1 mL) was added, and the mixture was stirred for 2 hr. After evaporating the solvent, the residue was dissolved in anhydrous pyridine (30 mL) under an argon atmosphere, mixed with cholesterol chloroformate (0.410 g), and the mixture was stirred overnight. The precipitated solid was collected by filtration, washed with tetrahydrofuran (50 mL) and dried to give a white solid (0.188 g).

This solid was dissolved in anhydrous chloroform (10 mL), and PEG-diamine (number average molecular weight of PEG: 2,000) (250 mg), triethylamine (100 µL), 1-(3-dimethylaminopropyl)-3-ethylcarbodiimide hydrochloride (150 mg), N-hydroxysuccinic acid (75 mg), and dimethylaminopyridine (10 mg) were added, and the mixture was stirred overnight. The precipitated solid was collected by filtration and washed with tetrahydrofuran (50 mL) to give a white solid (0.120 g).

The whole amount of the obtained solid was dissolved in anhydrous dichloromethane (30 mL) under an argon atmosphere, (1R,8S,9s)-bicyclo[6.1.0]non-4-yn-9-ylmethyl succinimidyl carbonate (20 mg), triethylamine (50 µL), 1-(3-dimethylaminopropyl)-3-ethylcarbodiimide hydrochloride (100 mg), N-hydroxysuccinic acid (30 mg), and dimethylaminopyridine (5 mg) were added, and the mixture was stirred overnight. The precipitated solid was collected by filtration and washed with tetrahydrofuran (30 mL) to give DiChol-PEG2k-BCN (0.058 g) as a white solid.

### Ligand having cell directivity

The lipid nanoparticles of the present invention are characterized by being modified with a ligand having cell directivity. As used herein, the "cell directivity" means having a higher affinity for one or more specific cells (including tissue and organ containing the cells) than for other cell types, as a result of which, lipid nanoparticles can be delivered more selectively to specific cells.

The ligand is not particularly limited as long as it is a substance having specific affinity for molecules expressed on the surface of target cells to which the lipid nanoparticles are to be delivered (for example, cells (e.g., cancer cells, etc.) into which a drug (preferably a nucleic acid) encapsulated in the lipid nanoparticles is introduced into the cells to exert a therapeutic effect on a disease). Examples thereof include peptide (including protein), antibody, nucleic acid (including oligonucleotide), aptamer, sugar, lipid, and the like. Preferred examples thereof include peptide, antibody, and the like.

Examples of the peptide having cell directivity include, but are not limited to, peptides containing RGD motif (e.g., GRGDS pentapeptide) that has affinity for integrin αᵥβ₃, which is highly expressed in the vascular system and some cancer cells, laminin α2 chain-derived peptide having affinity for dystroglycan highly expressed in muscle cells, and the like. Alternatively, polypeptides that are natural ligands for cell surface receptors such as cytokine, hormone, and the like can also be mentioned.

Antibodies having cell directivity include any antibodies against molecules expressed on the surface of target cells. Examples thereof include, but are not limited to, antibodies against CD3, CD28, CD4 (helper T cell) or CD8 (killer T cell) expressed in T cells, antibodies against CD19 expressed in B cells, and the like. Alternatively, antibodies used as existing antibody drugs or antibodies that recognize the same antigen as these can be used as ligands to target target cells expressing the antigen.

Antibodies used in the present invention may be complete antibody molecules, and, for example, any other antibody fragments such as single-stranded antibody (scFv), Fab, F(ab')₂, Fab', Fv, reduced antibody (rIgG), dsFv, sFv, diabody, triabody, and the like may be preferably used. In the present specification, unless otherwise specified, the "antibody" is used to include antibody fragments.

In addition, examples of the nucleic acid include ligands for Toll-like receptors, such as double-stranded RNA, CpG oligonucleotide, and the like, examples of the sugar include N-acetylgalactosamine having affinity for asialoglycoprotein receptor expressed on hepatocytes and the like, and examples of the lipid include various lipids (e.g., lysophosphatidylserine) which are natural ligands for G protein coupled receptor and the like.

The amount of the ligand is preferably 0.01 to 30 mol%, more preferably 0.01 to 10 mol%, further preferably 0.01 to 5 mol%, with respect to the total amount of lipids in the lipid nanoparticle, from the aspects of simultaneous achievement of the selectivity for target cells and preferred particle properties.

### Production method of lipid nanoparticles

The lipid nanoparticles of the present invention (i.e., lipid nanoparticles modified with ligand having cell directivity) can be produced by, for example,
producing lipid nanoparticles from a lipid material containing ionic lipid (1) and activated PEG lipid (preferably activated PEG lipid (2)) (hereinafter sometimes to be referred to as "the precursor of the present invention", and
binding the ligand having cell directivity to the obtained lipid nanoparticles.

The precursor of the present (i.e., lipid nanoparticles without ligand having cell directivity) invention can be produced by dispersing a lipid material containing ionic lipid (1) and activated PEG lipid (preferably activated PEG lipid (2)) in a suitable dispersion medium (for example, aqueous dispersion medium, alcoholic dispersion medium), and performing an operation to induce organization as necessary.

Examples of the "operation to induce organization" for producing the precursor of the present invention include methods known per se such as ethanol dilution method using a microchannel or vortex, simple hydration method, sonication, heating, vortex, ether injecting method, French press method, cholic acid method, Ca²⁺ fusion method, freeze-thaw method, reversed-phase evaporation method, and the like, preferably ethanol dilution method using a microchannel or vortex, further preferably ethanol dilution method using a microchannel. In the ethanol dilution method using a microchannel, for example, a dispersion containing lipid nanoparticles can be produced by mixing an acidic buffer containing a nucleic acid and an ethanol solution of a lipid by using NanoAssemblr (Precision NanoSystems). The dispersion produced by this method contains lipid nanoparticles and a dispersion medium (acidic buffer and ethanol). The dispersion medium (particularly ethanol) can be removed, the dispersion medium (particularly buffer) can be exchanged, and the like by operations such as ultrafiltration, dialysis, dilution, and the like.

By binding a ligand having cell directivity to the obtained precursor of the present invention, the lipid nanoparticles of the present invention can be produced. This bond can be formed by adding a ligand with cell directivity to a dispersion containing the precursor of the present invention. The concentration of the whole lipids of the precursor in the dispersion is preferably 1 to 100 mM, more preferably 1 to 50 mM, further preferably 1 to 30 mM.

The proportion of the ligand to be used is preferably 1 to 100 mol, more preferably 1 to 50 mol, further preferably 1 to 10 mol, per 1 mol of the activated PEG lipid (preferably activated PEG lipid (2)) in the precursor of the present invention. The reaction temperature for this binding is preferably 0 to 60°C, more preferably 0 to 40°C, further preferably 0 to 20°C, and the reaction time therefor is preferably 1 to 72 hr, more preferably 1 to 48 hr, further preferably 1 to 24 hr.

### Method for introducing nucleic acid-introducing agent and nucleic acid into cells

A nucleic acid can be introduced into a cell in vivo and/or in vitro by encapsulating the nucleic acid in the lipid nanoparticles of the present invention and contacting the lipid nanoparticles with the cell. Therefore, the present invention also provides a nucleic acid-introducing agent containing the lipid nanoparticles of the present invention, and a method for introducing a nucleic acid into cells by using the agent.

The nucleic acid-introducing agent of the present invention can introduce any nucleic acid into cells. Examples of the nucleic acid include, but are not limited to, DNA, RNA, chimera nucleic acid of RNA, DNA/RNA hybrid, and the like. While any single-stranded to triple-stranded nucleic acid can be used, it is preferably single-stranded or double-stranded. The nucleic acid may be a nucleotide having N-glycoside of purine or pyrimidine base, an oligomer having a non-nucleotide backbone (e.g., commercially available peptide nucleic acid (PNA) etc.), or an oligomer containing a special bond (said oligomer containing a nucleotide having a configuration permitting base pairing or attachment of base, which are found in DNA and RNA) and the like.

Furthermore, the nucleic acid may be, for example, a nucleic acid added with known modification, a nucleic acid with a label known in the field, a nucleic acid with a cap, a methylated nucleic acid, a nucleic acid with one or more natural nucleotides substituted by an analog, a nucleic acid with modified nucleotide, a nucleic acid having a non-charge bond (e.g., methylphosphonate, phosphotriester, phosphoramidate, carbamate, and the like), a nucleic acid having a charged bond or sulfur-containing bond (e.g., phosphorothioate, phosphorodithioate, and the like), a nucleic acid having a side chain group such as protein (e.g., nuclease, nuclease inhibitor, toxin, antibody, signal peptide, poly-L-lysine, and the like), sugar (e.g., monosaccharide and the like), and the like, a nucleic acid containing an intercalating compound (e.g., acridine, psoralen, and the like), a nucleic acid containing a chelate compound (e.g., metal, radioactive metal, boron, oxidative metal, and the like), a nucleic acid containing an alkylating agent, or a nucleic acid containing a modified bond (e.g., α anomer-type nucleic acid and the like).

The type of the DNA that can be used in the present invention is not particularly limited, and can be selected as appropriate according to the purpose of use. Examples of the DNA include plasmid DNA, cDNA, antisense DNA, chromosomal DNA, PAC, BAC, CpG oligosaccharide, and the like. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. Examples of the RNA include siRNA, miRNA, shRNA, antisense RNA, messenger RNA (mRNA), single-stranded RNA genome, double-stranded RNA genome, RNA replicon, transfer RNA, ribosomal RNA, and the like, with preference given to siRNA, miRNA, shRNA, mRNA, antisense RNA, and RNA replicon.

The nucleic acid used in the present invention is' preferably purified by a method generally used by those of ordinary skill in the art.

The nucleic acid-introducing agent of the present invention encapsulating a nucleic acid can be administered in vivo for the purpose of, for example, prevention and/or treatment of diseases. Therefore, the nucleic acid to be used in the present invention is preferably one having a preventive and/or therapeutic activity against a given disease (prophylactic/therapeutic nucleic acid). Examples of such nucleic acid include nucleic acids used for so-called gene therapy, and the like.

The nucleic acid-introducing agent of the present invention encapsulating a nucleic acid can be used as a drug delivery system for selectively delivering a nucleic acid and the like into a particular cell, and is useful for, for example, DNA vaccines by introducing antigen gene into dendritic cells, gene therapy drugs for tumor, nucleic acid pharmaceutical products that suppress expression of target genes by utilizing RNA interference, and the like.

The particle size of the lipid nanoparticle encapsulating a nucleic acid is not particularly limited, and is preferably 10 nm to 500 nm, more preferably 30 nm to 300 nm. The particle size can be measured by using a particle size distribution measuring device such as Zetasizer Nano (Malvern) or the like. The particle size of the lipid nanoparticle encapsulating a nucleic acid can be appropriately adjusted by the production method thereof.

The surface charge (zeta potential) of the lipid nanoparticles encapsulating a nucleic acid is not particularly limited and preferably -15 to +15 mV, more preferably -10 to +10 mV. In conventional transgene, particles with positively charged surfaces have been mainly used. This is useful as a method for promoting electrostatic interactions with heparin sulfate on the negatively-charged cell surface to enhance uptake into cells. However, the positive surface potential may suppress (a) nucleic acid release from the carrier due to the interaction with a nucleic acid to be delivered in the cell, or (b) protein synthesis due to the interaction between mRNA and a nucleic acid to be delivered. This problem can be solved by adjusting the surface potential (zeta potential) to fall within the above-mentioned range. The surface potential (zeta potential) can be measured using a zeta potential measuring apparatus such as Zetasizer Nano or the like. The surface potential (zeta potential) of the lipid nanoparticles can be adjusted by the composition of the constituent components of the lipid nanoparticles.

The method of contacting the ucleic acid-introducing agent (lipid nanoparticles) of the present invention encapsulating the nucleic acid with the cell in vitro is specifically explained below.

The cells are suspended in a suitable medium several days before contact with the nucleic acid-introducing agent of the present invention encapsulating a nucleic acid, and cultured under appropriate conditions. At the time of contact with the nucleic acid-introducing agent of the present invention, the cells may or may not be in a proliferative phase.

The culture medium on contact between the nucleic acid-introducing agent of the present invention encapsulating a nucleic acid and the cell may be a serum-containing medium or a serum-free medium, wherein the serum concentration of the medium is preferably not more than 30 wt%, more preferably not more than 20 wt%. When the medium contains excess protein such as serum and the like, the contact between the nucleic acid-introducing agent of the present invention and the cell may be inhibited.

The cell density on contact between the nucleic acid-introducing agent of the present invention encapsulating a nucleic acid and the cell is not particularly limited, and can be appropriately determined in consideration of the kind of the cell and the like. It is generally within the range of 1×10⁴ to 1×10⁷ cells/mL.

For example, a dispersion of the nucleic acid-introducing agent of the present invention enclosing the nucleic acid is added to the cells. The amount of the dispersion to be added is not particularly limited, and can be appropriately determined in consideration of the cell number and the like. The concentration of the lipid nanoparticles contained in the nucleic acid-introducing agent of the present invention to be contacted with the cells is not particularly limited as long as the desired introduction of the nucleic acid into the cells can be achieved. The concentration of the lipid in the dispersion is generally 1 to 100 nmol/ml, preferably 10 to 50 nmol/ml, and the concentration of the nucleic acid in the dispersion is generally 0.01 to 100 µg/ml, preferably 0.1 to 10 µg/ml.

After the aforementioned dispersion is added to cells, the cells are cultured. The temperature, humidity and CO₂ concentration during culturing are appropriately determined in consideration of the kind of the cell. When the cell is a mammal-derived cell, generally, the temperature is about 37°C, relative humidity is about 95% and CO₂ concentration is about 5% by volume. While the culture period can also be appropriately determined in consideration of the conditions such as the kind of the cell and the like, it is generally a range of 0.1 to 76 hr, preferably a range of 0.2 to 24 hr, more preferably a range of 0.5 to 12 hr. When the above-mentioned culture time is too short, the nucleic acid is not sufficiently introduced into the cells, and when the culture time is too long, the cells may become weak.

By the above-mentioned culture, the nucleic acid is introduced into cells. The culture is further continued preferably by exchanging the medium with a fresh medium, or adding a fresh medium to the medium. When the cell is a mammal-derived cell, the fresh medium preferably contains a serum or nutrition factor.

A nucleic acid can be introduced into cells not only outside the body (in vitro) but also in the body (in vivo) by using the nucleic acid-introducing agent of the present invention. That is, by administration of the nucleic acid-introducing agent of the present invention encapsulating the nucleic acid to a subject, the nucleic acid-introducing agent of the present invention reaches and contacts with the target cells, and the nucleic acid encapsulated in the nucleic acid-introducing agent of the present invention is introduced into the cells in vivo. The subject to which the nucleic acid-introducing agent of the present invention encapsulating the nucleic acid can be administered is not particularly limited and, for example, vertebrates such as mammals (e.g., human, monkey, mouse, rat, hamster, bovine, etc.), birds (e.g., chicken, ostrich, etc.), amphibia (e.g., frog etc.), fishes (e.g., zebrafish, rice-fish, etc.), and the like, invertebrates such as insects (e.g., silk moth, moth, Drosophila, etc.) and the like, plants, and the like can be mentioned. The subject of administration of the nucleic acid-introducing agent of the present invention encapsulating the nucleic acid is preferably human or other mammal.

The kind of the target cell is not particularly limited, and a nucleic acid can be introduced into cells in various tissues (e.g., liver, kidney, pancreas, lung, spleen, heart, blood, muscle, bone, brain, stomach, small intestine, large intestine, skin, adipose tissue, lymph node, tumor, etc.) by using the nucleic acid-introducing agent of the present invention encapsulating a nucleic acid.

The administration method of the nucleic acid-introducing agent of the present invention encapsulating a nucleic acid to a target (e.g., vertebrate, invertebrate, and the like) is not particularly limited as long as the nucleic acid-introducing agent of the present invention reaches and contacts with the target cells, and the nucleic acid encapsulated in the nucleic acid-introducing agent of the invention can be introduced into the cell, and an administration method known per se (e.g., oral administration, parenteral administration (e.g., intravenous administration, intramuscular administration, topical administration, transdermal administration, subcutaneous administration, intraperitoneal administration, spray, etc.), etc.) can be appropriately selected in consideration of the kind of the nucleic acid to be introduced, the kind and the site of the target cell, and the like. The dose of the nucleic acid-introducing agent of the present invention is not particularly limited as long as the introduction of the nucleic acid into the cells can be achieved, and can be appropriately selected in consideration of the kind of the subject of administration, administration method, the kind of the nucleic acid to be introduced, the kind and the site of the target cell and the like. The lipid nanoparticles of the present invention can also be used for introducing a compound other than nucleic acid into cells.

The nucleic acid-introducing agent of the present invention encapsulating a nucleic acid can be formulated according to a conventional method.

When the nucleic acid-introducing agent of the present invention is provided as a reagent for studies, the nucleic acid-introducing agent of the present invention may be provided as a sterile dispersion of the lipid nanoparticles of the present invention and water or physiologically acceptable other dispersion medium (e.g., water-soluble dispersion medium (e.g., malic acid buffer, etc.), organic dispersion medium (e.g., ethanol, methanol, DMSO, tert-butanol, and the like), or a mixture of aqueous dispersion medium and organic dispersion medium, etc.), or may be provided as a nucleic acid-introducing agent without a dispersion medium. The nucleic acid-introducing agent of the present invention may appropriately contain physiologically acceptable additive (e.g., excipient, vehicle, preservative, stabilizer, binder, etc.), which are known per se.

When the nucleic acid-introducing agent of the present invention is provide as a medicament, the nucleic acid-introducing agent of the present invention may be provided as an oral preparation (e.g., tablet, capsule, etc.) or a parenteral agent (e.g., injection, spray, etc.) obtained by mixing the lipid nanoparticles of the present invention and pharmaceutically acceptable known additives (e.g., carrier, flavor, excipient, vehicle, preservative, stabilizer, binder, etc.), or may be provided as a nucleic acid-introducing agent free of known additives. The nucleic acid-introducing agent of the present invention as a medicament is preferably a parenteral agent (more preferably an injection). The nucleic acid-introducing agent of the present invention may be a preparation for adults or for children.

### [Example]

The contents of the present invention are further explained in detail in the following by using Examples and the like, but the present invention is not limited in any way by the Examples and the like.

In the following Examples and the like, ionic lipid (1) is shown by the name listed in the aforementioned Table. The abbreviations used in the following Examples and the like each mean the following.
Azido-PEG4-NHS: N-succinimidyl 15-azido-4,7,10,13-tetraoxa pentadecanoate
Chol: cholesterol
Chol-PEG2k-BCN: cholesteryl carbamoyl, 1R,8S,9s)-bicyclo[6.1.0]non-4-yn-9-ylmethyl, polyethylene glycol (number average molecular weight (Mn) of PEG: 2000),
Cy5-Alkyne: 3,3-dimethyl,1-1-[6-oxo-6-(prop-2-ylamino)hexyl]-2-[(1E,3E,5E)-5-(1,3,3-trimethylindolin-2-ylidene)penta-1,3-dienyl]-3H-indolium chloride
DiChol-PEG2k-BCN: N-(2-polyethylene glycol (number average molecular weight (Mn) of PEG: 2000))-3,5-bis(cholesterylamino)pentanamide
DiD: 1,1'-dioctadecyl-3,3,3',3'-tetramethylindocarbocyanine perchlorate
DMG-PEG2k: 1,2-dimyristoyl-rac-glycerol, methoxypolyethylene glycol (number average molecular weight (Mn) of PEG: 2000) DOPC: 1,2-dioleoyl-sn-glycero-3-phosphocholine
DSG-PEG2k-DBCO: 1,2-distearoyl-rac-glycerol, dibenzocyclooctyne thoxypolyethylene glycol (number average molecular weight (Mn) of PEG: 2000), N-(1R,8S,9s)-bicyclo[6.1.0]non-4-yn-9-ylmethyloxycarbonyl-1,8-diamino-3,6-dioxaoctane
DSPE-PEG2k-DBCO: 1,2-distearoyl-rac-glycerol phosphoethanolamine, dibenzocyclooctyne thoxypolyethylene glycol (number average molecular weight (Mn) of PEG: 2000), N-(1R,8S,9s)-bicyclo[6.1.0]non-4-yn-9-ylmethyloxycarbonyl-1,8-diamino-3,6-dioxaoctane
FBS: fetal bovine serum
MES: 2-morpholinoethanesulfonic acid
PBS: phosphate buffered saline

### [Reference Example 1] Fragmentation of CD3-IgG and introduction of reaction substrate

### 1. Fragmentation and purification of antibody

Anti-CD3 IgG antibody (CD3-IgG, source: manufactured by BioXCell) was desalted using a zeba-spin desalting column and replaced with sodium acetate buffer (20 mM, pH 4.5). The CD3-IgG after replacement was stirred at 37°C for 3 days at a ratio of CD3-IgG:pepsin=10 mg:0.125 mg to perform fragmentation. Separation from pepsin was performed by passing the fragment solution through an empty column. For the purpose of purification and buffer replacement, the fragment solution passed through the column was transferred to Amicon Ultra15 (MWCO: 50 kDa), diluted to 14 mL using sodium carbonate buffer (100 mM, pH 9.0), and centrifuged (25°C, 1000 g, 5 min) was repeated to ultrafiltrate and concentrate to about 500 µL. Thereafter, the volume was increased to 14 mL using sodium carbonate buffer (100 mM, pH 9.0), and centrifugation was repeated again (25°C, 1000 g, 5 min) to concentrate to 500 µL. Finally, the volume was increased to 1 mL using sodium carbonate buffer (100 mM, pH 9.0).

### 2. Introduction of reaction substrate

Protein concentration and protein amount were calculated by measuring the absorbance of the fragment solution at 280 nm using Nanodrop one (Thermo Scientific). 4 equivalents of Azido-PEG4-NHS were added to the fragment solution, and the mixture was shaken at room temperature for 2 hr and at 4°C overnight to introduce the reaction substrate. After introducing the reaction substrate, unreacted reagents were removed from the fragment solution using a zeba-spin desalting column and the solution was replaced with ultrapure water.

### 3. Confirmation of reaction substrate introduction

An amount equivalent to 100 µg of the fragment solution after introducing the substrate was taken into a light-shielding Eppendorf tube, 10 µL of ascorbic acid (20 mM), 10 µL of copper sulfate aqueous solution (20 mM), 2 µL of Cy5-Alkyne (10 mg/mL), and finally ultrapure water were added to increase the volume to 100 µL, and the mixture was reacted at room temperature for 2 hr. After the reaction, the reaction solution was transferred to a dialysis membrane with a cut-off molecular weight of 3.5 kDa, and dialysis was performed overnight. After dialysis, the amount of protein was calculated using the BCA method, and the amount of Cy5 modification was calculated based on fluorescence. The number of introduced reaction substrates was determined by dividing the amount of Cy5 modification by the amount of protein.

### [Example 1] Production of ligand-modified and mRNA-encapsulating LNP

### 1. Production of mRNA-encapsulating LNP using microchannel

### (1) Production of lipid ethanol solution

For the preparation of an ethanol solution of lipids, 10 mM O-Ph-P4C2, 5 mM phospholipid, and 5 mM cholesterol were mixed in an Eppendorf tube at the desired ratio so that the total lipid amount was 800 nmol. 1 mM DMG-PEG2k ethanol solution was added in an amount equivalent to 1.5% of the total lipid amount, and activated PEG lipid was further added in an amount equivalent to 1% of the total lipid amount, and ethanol was added so that the total amount was 360 µL.

### (2) Production of acidic buffer solution of nucleic acid

For the preparation of a nucleic acid acidic buffer solution, the mRNA solution (concentration varied depending on the efficiency of in vitro translation, but it was generally 0.6 to 0.8 µg/µL) was measured into a 5 mL tube to give 6.6 µg, and acidic malate buffer (20 mM, pH 3.0, containing 30 mM NaCl) was added to make the total amount 990 µL.

### (3) Production of mRNA-encapsulating LNP using microchannel

An acidic buffer solution of nucleic acid and an ethanol solution of lipid were each weighed into a syringe. Using NanoAssmblr (registered trademark) ultra-high-speed nanomedicine production device (manufactured by Nepa Gene Co., Ltd.), LNP production was performed using the nucleic acid solution at a flow rate of 3 mL/min and the lipid solution at 1 mL/min, and 0.8 mL was collected into a 15 mL tube. After adding 3200 µL of 20 mM MES (adjusted to pH 6.5 with NaOH) to the 15 mL tube, the tube was transferred to Amicon Ultra 4, centrifuged repeatedly (25°C, 1000 g, 5 min), and ultrafiltered to about 500 µL and concentrated. Thereafter, the volume was increased to 4 mL using PBS, centrifuged repeatedly (25°C, 1000 g, 5 min) again, and ultrafiltered and concentrated to about 500 µL. Thereafter, the volume was increased to 4 mL using acidic malate buffer (20 mM, pH 4.0, containing 30 mM NaCl), and centrifugation was repeated again (25°C, 1000 g, 5 min) to reduce the volume to about 250 µL by ultrafiltration and concentration. Finally, the mixture was diluted to the desired lipid concentration using acidic malate buffer (20 mM, pH 4.0, containing 30 mM NaCl) to produce an mRNA-encapsulating LNP dispersion.

### 2. Modification of ligand to LNP

To the produced mRNA-encapsulating LNP dispersion were added the ligand at an equimolar amount to the activated PEG lipid, the volume was increased to 750 µL using acidic malate buffer (20 mM, pH 4.0, containing 30 mM NaCl), and the mixture was reacted overnight at 4°C. Thereafter, the mixture was transferred to Amicon Ultra 4, increased to 4 mL using PBS, centrifuged repeatedly (25°C, 1000 g, 5 min), and ultrafiltered and concentrated to about 500 µL. Thereafter, the volume was increased to 4 mL using PBS, centrifuged repeatedly (25°C, 1000 g, 5 min) again, and ultrafiltered and concentrated to about 250 µL. Finally, the mixture was increased to the desired lipid concentration using PBS to produce a ligand-modified and mRNA-encapsulating LNP dispersion.

### [Example 2] Production of T-cell-directional ligand-modified and mRNA-encapsulating LNP

mRNA-encapsulating LNP (molar ratio of O-Ph-P4C2:DOPC:Chol = 52.5:7.5:40, and further using 1.5 mol% DMG-PEG2k, 1 mol% DSPE-PEG2k-DBCO (manufactured by Avanti Polar Lipids), and 0.5 mol% DiD as an index of uptake, each to the total of O-Ph-P4C2, DOPC, and Chol) was produced by the method described in [Example 1]. Thereafter, LNP was modified by the method of [Example 1] using F(ab')2 produced from an anti-CD3-IgG antibody as a ligand to prepare ligand-modified and mRNA-encapsulating LNP.

### [Experimental Example 1] Evaluation of LNP uptake ability of splenic T cells in vivo (FACS analysis)

The ligand-modified and mRNA-encapsulating LNP produced in Example 2 was diluted with PBS to an mRNA concentration of 2.5 µg/mL. The diluted ligand-modified and mRNA-encapsulating LNP was intravenously administered to 6-week-old female C57/BL6J mouse at a dose of 10 µL per 1 g of body weight (mRNA dose: 0.025 mg/kg). One day after administration, the spleen was collected from the mouse and collected in a petri dish containing 3 mL of RPMI-1640 (containing 1% FBS). The spleen cells were filtered out using forceps and suspended in a 5 mL syringe. The entire volume was transferred to a 15 mL centrifuge tube via a 70 µm cell strainer. After centrifugation (4°C, 500 g, 5 min), the supernatant was removed, Red Blood Cell Lysing Buffer (1 mL) was added to suspend the cells, and the mixture was allowed to stand at room temperature for 5 min to perform hemolytic treatment. Thereafter, the mixture was diluted 5 times using FACS buffer (PBS containing 0.5% bovine serum albumin (BSA) and 0.1% NaN₃), centrifuged (4°C, 500 g, 5 min), and the supernatant was removed. After washing twice with 5 mL of FACS buffer (PBS containing 0.5% bovine serum albumin (BSA), 0.1% NaN₃), the cells were counted (LUNA), 1×10⁶ cells were dispensed, and Fc blocking was performed. For Fc blocking, CD16/32 antibody (source: BioLegend) was added in the amount recommended by the manufacturer, and incubated on ice for 10 min or more. Brilliant Violet 605 anti-mouse CD45, Brilliant Violet 510 anti-mouse CD19, Brilliant Violet 421 anti-mouse CD3ε, PE anti-mouse CD4 and Alexa Fluor 488 anti-mouse CD8α (source: BioLegend) were added in the manufacturer's recommended amounts, and fluorescent labeling of cell surface antigens was performed by incubating on ice for 30 min. With 5 min remaining for antibody staining, 7-AAD Viability Staining Solution (source: BioLegend) was added in the manufacturer's recommended amount to perform dead cell staining. After washing the cells twice with FACS buffer, they were resuspended in FACS buffer (200 µL), subsets were classified using a Novocyte flow cytometer (ACEA Biosciences), and DiD fluorescence was analyzed for each subset.

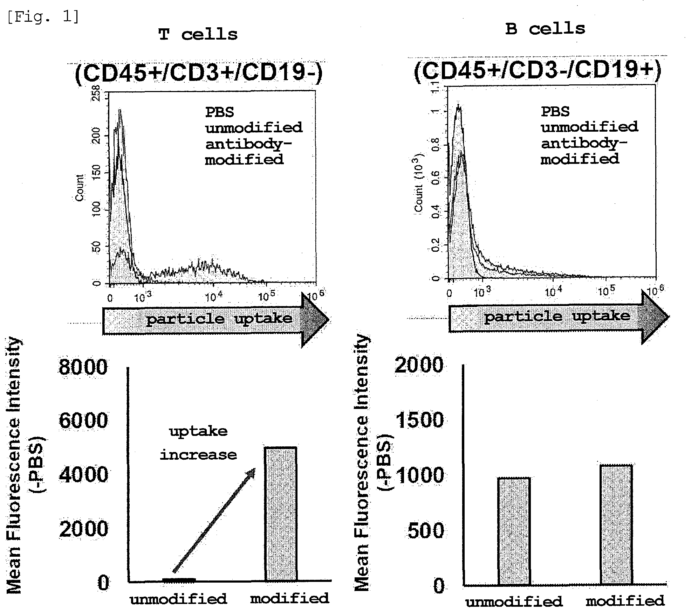

The results are shown in Fig. 1. The "unmodified" and "antibody-modified" in Fig. 1 indicate the results using unmodified LNP or antibody-modified LNP, respectively, and "PBS" in Fig. 1 indicates the results not using unmodified LNP or antibody-modified LNP. By modifying LNP with an anti-CD3 antibody fragment, selective uptake into T cells was observed.

### [Experimental Example 2] Evaluation of LNP uptake ability in vivo (microscope observation)

### 1. LNP administration and isolation of splenic T cells

The ligand-modified and mRNA-encapsulating LNP produced by the method described in Example 2 was diluted with PBS to an mRNA concentration of 2.5 µg/mL. The diluted ligand-modified and mRNA-encapsulating LNP was intravenously administered to 6-week-old female C57/BL6J mouse at a dose of 10 µL per 1 g of body weight (mRNA dose: 0.025 mg/kg). One day after administration, the spleen was collected from the mouse and a cell suspension was produced by the method described in [Experimental Example 1]. 3×10⁶ cells were dispensed and Fc blocking was performed. For Fc blocking, CD16/32 antibody (mentioned above) was added in the amount recommended by the manufacturer, and incubated on ice for 10 min or more. Brilliant Violet 510 anti-mouse CD45 and Brilliant Violet 421 anti-mouse CD3ε (mentioned above) were added in the amounts recommended by the manufacturer, and fluorescent labeling of cell surface antigens was performed by incubating on ice for 30 min. After washing the cells twice with FACS buffer, they were resuspended in FACS buffer (500 µL), and 2×10⁵ T cells were isolated using BD FACSMelody^{™} cell sorter (Becton, Dickinson and Company).

### 2. Encapsulation and microscopic observation

The isolated T cells were centrifuged (4°C, 500 g, 5 min) and then concentrated by removing the supernatant. ProLong Diamond anti-fading mounting medium (10 µL) and the concentrated T cells (10 µL) were mixed, 10 µL was dropped onto a glass plate, and a cover glass was placed for encapsulation. After standing overnight at 4°C, DiD fluorescence was observed using an LSM780 confocal laser microscope (Zeiss).

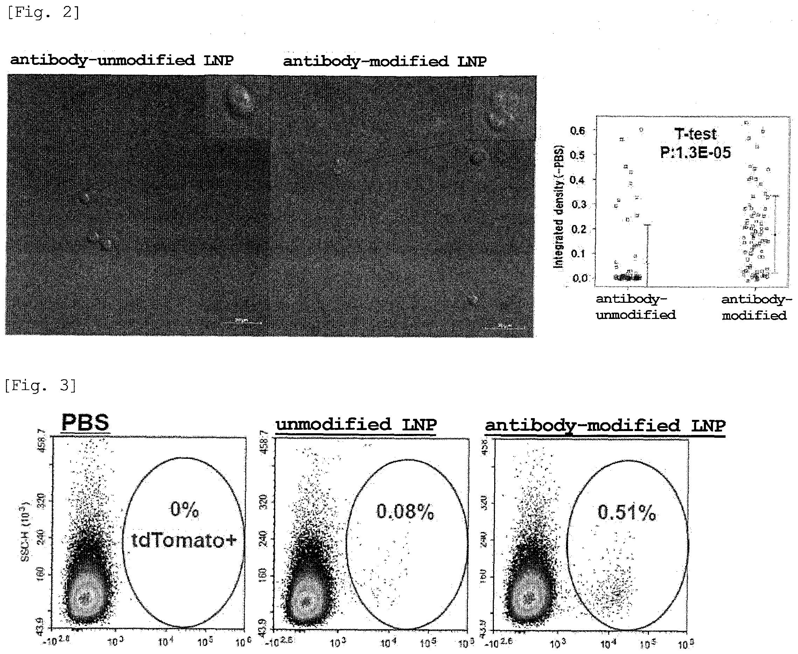

The results are shown in Fig. 2., In Fig. 2, the "unmodified (LNP)" or "antibody-modified (LNP)" indicate the results using unmodified LNP or antibody-modified LNP, respectively. Microscopic observation also confirmed that LNP uptake into T cells significantly increased by modification with an anti-CD3 antibody fragment.

### [Experimental Example 3] Evaluation of LNP uptake ability in vivo (reporter gene expression)

### 1. Production of LNP

LNP encapsulating mRNA expressing Cre recombinase (molar ratio of O-Ph-P4C2:DOPC:Chol = 52.5:7.5:40, and further using 1.5 mol% of DMG-PEG2k and 1 mol% of DSPE-PEG2k-DBCO with respect to the total of O-Ph-P4C2, DOPC, and Chol) was produced by the method described in [Example 2]. Thereafter, LNP was modified by the method of [Example 2] and using F(ab')2 produced from an anti-CD3-IgG antibody as a ligand to prepare ligand-modified and mRNA-encapsulating LNP.

### 2. Evaluation of LNP uptake ability of splenic T cells in vivo

The ligand-modified and mRNA-encapsulating LNP produced in the above-mentioned 1. was diluted with PBS to an mRNA concentration of 5 µg/mL. The diluted ligand-modified and mRNA-encapsulating LNP was intravenously administered to 6-week-old female Ai14 mouse (loxP-stop codon-loxP-tdTomato gene was integrated into ROSA26 locus, and stop codon was excised in the presence of Cre recombinase to express tdTomato reporter; purchased from The Jackson Laboratory) at a dose of 10 µL per 1 g of body weight (mRNA dose: 0.05 mg/kg). Three days after administration, the spleen was collected from the mouse and a suspension was produced by the method described in [Experimental Example 1]. 2×10⁶ cells were dispensed, and Fc blocking was performed. For Fc blocking, CD16/32 antibody (mentioned above) was added in the amount recommended by the manufacturer, and incubated on ice for 10 min or more. Brilliant Violet 605 anti-mouse CD45, Brilliant Violet 510 anti-mouse CD19, and Brilliant Violet 421 anti-mouse CD3s (mentioned above) were added in the manufacturer's recommended amounts, and fluorescent labeling of cell surface antigens was performed by incubating on ice for 30 min. With 5 min remaining for antibody staining, 7-AAD Viability Staining Solution (mentioned above) was added in the manufacturer's recommended amount to perform dead cell staining. After washing the cells twice with FACS buffer, they were resuspended in FACS buffer (400 µL), subsets were classified using a Novocyte flow cytometer (ACEA Biosciences), and tdTomato expression in T cells (CD45+/CD3+/CD19-) was analyzed.

The results are shown in Fig. 3. In Fig. 3, "PBS" indicates the results not using unmodified LNP or antibody-modified LNP. In LNP modified with anti-CD3 antibody fragment, the number of tdTomato-expressing cells increased as compared with unmodified LNP, and it was clarified that antibody-modified LNP improves mRNA delivery efficiency into T cells.

### [Example 3] Production of mRNA-encapsulating LNP using novel activated PEG lipid

### (1) Production of lipid ethanol solution

For the preparation of an ethanol solution of lipids, 10 mM O-Ph-P4C2, 5 mM phospholipid, and 5 mM cholesterol were mixed in an Eppendorf tube at the desired ratio so that the total lipid amount was 800 nmol. 1 mM DMG-PEG2k ethanol solution was added in an amount equivalent to 1.5 mol% of the total lipid amount, and activated PEG lipid was further added in an amount equivalent to 1 mol% of the total lipid amount, and ethanol was added so that the total amount was 360 µL. As the activated PEG lipid, DSPE-PEG2k-DBCO (manufactured by Avanti Polar Lipids), DSG-PEG2k-DBCO, Chol-PEG2k-BCN, or DiChol-PEG2k-BCN was used. Among these, DSG-PEG2k-DBCO, Chol-PEG2k-BCN, or DiChol-PEG2k-BCN used was synthesized in the same manner as in Synthetic Example described in [Description of Embodiments] .

### (2) Production of acidic buffer solution of nucleic acid

For the preparation of a nucleic acid acidic buffer solution, the mRNA solution (concentration varied depending on the efficiency of in vitro translation, but it was generally 0.6 to 0.8 µg/µL) was measured into a 5 mL tube to give 6.6 µg, and acidic malate buffer (20 mM, pH 3.0, containing 30 mM NaCl) was added to make the total amount 990 µL.

### (3) Production of LNP using microchannel

An acidic buffer solution of nucleic acid and an ethanol solution of lipid were each weighed into a syringe. Using NanoAssmblr (registered trademark) ultra-high-speed nanomedicine production device, LNP production was performed using the nucleic acid solution at a flow rate of 3 mL/min and the lipid solution at 1 mL/min, and 0.8 mL was collected into a 15 mL tube. After adding 3200 µL of 20 mM MES (adjusted to pH 6.5 with NaOH) to the 15 mL tube, the tube was transferred to Amicon Ultra 4, centrifuged repeatedly (25°C, 1000 g, 5 min), and ultrafiltered to about 500 µL and concentrated. Thereafter, the volume was increased to 4 mL using PBS, centrifuged repeatedly (25°C, 1000 g, 5 min) again, and ultrafiltered and concentrated to about 500 µL. Thereafter, the volume was increased to 4 mL using PBS, centrifuged repeatedly (25°C, 1000 g, 5 min) again, and ultrafiltered and concentrated to about 250 µL. Finally, the mixture was diluted to the desired lipid concentration using PBS to produce an mRNA-encapsulating LNP.

### [Experimental Example 4] Measurement of particle size and surface potential of mRNA-encapsulating LNP

The average particle size (number-based) and zeta potential of the mRNA-encapsulating LNP produced in Example 3 were measured by dynamic light scattering. For the obtained LNP, the average particle size, polydispersity index (hereinafter indicated as "PdI"), zeta potential, the ratio of mRNA contained in the solution after production to mRNA used during particle production (hereinafter indicated as "recovery rate"), and the ratio of mRNA loaded on the particles to the total mRNA in the solution after particle production (hereinafter indicated as "encapsulation rate") are shown in Table 2.

**[Table 2]**

| activated PEG lipid used | average particle size (nm) | PdI | zeta potential (mV) | recovery rate (%) | encapsulation rate (%) |
|---|---|---|---|---|---|
| DSPE-PEG2k-DBCO | 43.6 | 0.34 | -8.8 | 104.0 | 88.5 |
| DSG-PEG2k-DBCO | 51.6 | 0.19 | -6.2 | 103.3 | 92.9 |
| Chol-PEG2k-BCN | 53.7 | 0.19 | -9.7 | 83.9 | 76.4 |
| DiChol-PEG2k-BCN | 71.0 | 0.13 | -7.9 | 83.8 | 76.3 |

### [Experimental Example 5] Evaluation of in vivo LNP uptake ability (FACS analysis)

### 1. Production of ligand-modified and mRNA-encapsulating LNP

mRNA-encapsulating LNP (molar ratio of O-Ph-P4C2:DOPC:Chol = 52.5:7.5:40, and further using 1.5 mol% of DMG-PEG2k, 1 mol% of activated PEG lipid, and 0.5 mol% of DiD as an index of uptake, each with respect to the total of O-Ph-P4C2, DOPC, and Chol) was produced by the method described in [Example 2]. Thereafter, LNP was modified by the method of [Example 2] and using F(ab')2 produced from an anti-CD3-IgG antibody as a ligand to prepare ligand-modified and mRNA-encapsulating LNP. As the activated PEG lipid, DSPE-PEG2k-DBCO, DSG-PEG2k-DBCO, Chol-PEG2k-BCN, or DiChol-PEG2k-BCN was used.

### 2. Evaluation of LNP uptake ability of splenic T cells in vivo

The ligand-modified and mRNA-encapsulating LNP produced in the above-mentioned 1. was diluted with PBS to an mRNA concentration of 2.5 µg/mL. The diluted ligand-modified and mRNA-encapsulating LNP was intravenously administered to 6-week-old female C57/BL6J mouse at a dose of 10 µL per 1 g of body weight (mRNA dose: 0.025 mg/kg). One day after administration, the spleen was collected from the mouse and a cell suspension was produced by the method described in [Experimental Example 1]. 1×10⁶ cells were subjected to Fc blocking and antibody staining by the methods of [Experimental Example 1], subsets were classified using a Novocyte flow cytometer (ACEA Biosciences), and uptake of various LNPs into T cells (CD45+/CD3+/CD19-) was analyzed using DiD fluorescence as an index.

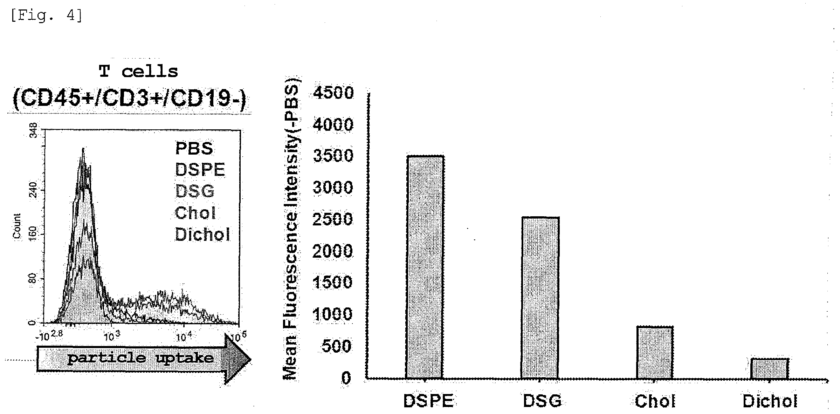

The results are shown in Fig. 4. In Fig. 4, "DSPE", "DSG", "Chol" or "DiChol" indicates the results using DSPE-PEG2k-DBCO, DSG-PEG2k-DBCO, Chol-PEG2k-BCN, or DiChol-PEG2k-BCN, respectively. In particular, when DSPE-PEG2k-DBCO or DSG-PEG2k-DBCO was used as the activated PEG lipid, LNP uptake into T cells remarkably increased.

### [Experimental Example 6] Evaluation of LNP uptake ability of peripheral blood cells in vivo

Each ligand-modified and mRNA-encapsulating LNP produced by the method described in Experimental Example 5, 1. was diluted with PBS to an mRNA concentration of 2.5 µg/mL. The diluted ligand-modified and mRNA-encapsulating LNP was intravenously administered to 6-week-old female C57/BL6J mouse at a dose of 10 µL per 1 g of body weight (mRNA dose: 0.025 mg/kg). One day after administration, 20 µL of blood sample was collected from the mouse tail vein, Red Blood Cell Lysing Buffer (1 mL) was added to suspend the cells, and the mixture was stirred at room temperature for 10 min to perform hemolytic treatment. Thereafter, centrifugation (4°C, 500 g, 5 min) was performed and the supernatant was removed. After suspending again with 1 mL of FACS buffer (PBS containing 0.5% bovine serum albumin (BSA), 0.1% NaN₃), centrifugation (4°C, 500 g, 5 min) was performed and the supernatant was removed. CD16/32 antibody (mentioned above) was added in the amount recommended by the manufacturer, and incubated on ice for 10 min or more for Fc blocking. Brilliant Violet 605 anti-mouse CD45, Brilliant Violet 510 anti-mouse CD19, Brilliant Violet 421 anti-mouse CD3ε, PE anti-mouse CD4 and Alexa Fluor 488 anti-mouse CD8α (mentioned above) were added in the manufacturer's recommended amounts, and fluorescent labeling of cell surface antigens was performed by incubating on ice for 30 min. With 5 min remaining for antibody staining, 7-AAD Viability Staining Solution (mentioned above) was added in the manufacturer's recommended amount to perform dead cell staining. After washing the cells twice with FACS buffer, they were resuspended in FACS buffer (200 µL), subsets were classified using a Novocyte flow cytometer (ACEA Biosciences), and uptake of various LNPs into T cells (CD45+/CD3+/CD19-) was analyzed using DiD fluorescence as an index.

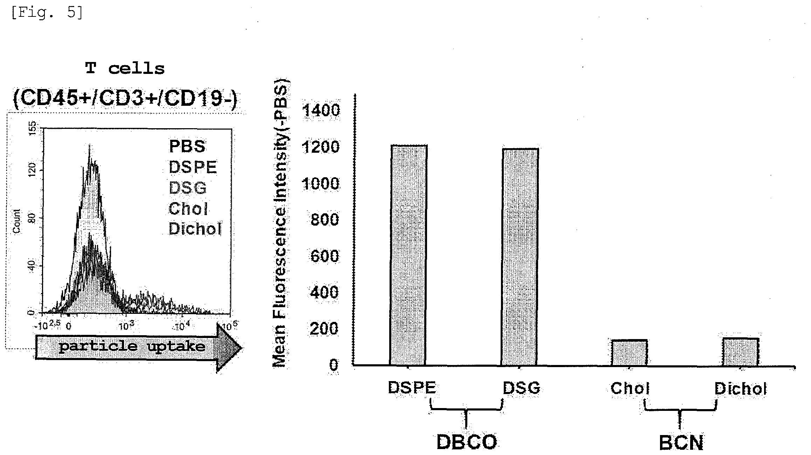

The results are shown in Fig. 5. In Fig. 5, "DSPE", "DSG", "Chol" or "DiChol" indicates the results using DSPE-PEG2k-DBCO, DSG-PEG2k-DBCO, Chol-PEG2k-BCN, or DiChol-PEG2k-BCN, respectively. In particular, similar to splenic T cells, when DSPE-PEG2k-DBCO or DSG-PEG2k-DBCO was used as the activated PEG lipid, LNP uptake into peripheral blood T cells remarkably increased.

### [Experimental Example 7] Evaluation of LNP uptake ability in vivo

### 1. Production of ligand-modified and mRNA-encapsulating LNP

In the same manner as in Experimental Example 3, 1., LNP encapsulating mRNA expressing Cre recombinase (molar ratio of O-Ph-P4C2:DOPC:Chol = 52.5:7.5:40, and further using 1.5 mol% of DMG-PEG2k and 1 mol% of activated PEG lipid with respect to the total of O-Ph-P4C2, DOPC, and Chol) was produced by the method described in [Example 2]. Thereafter, the LNP was modified by the method of [Example 2] and using F(ab')2 produced from an anti-CD3-IgG antibody as a ligand to prepare ligand-modified and mRNA-encapsulating LNP. As the activated PEG lipid, DSPE-PEG2k-DBCO or DSG-PEG2k-DBCO was used.

### 2. Evaluation of LNP uptake ability of splenic T cells in vivo

The produced ligand-modified and mRNA-encapsulating LNP was diluted with PBS to an mRNA concentration of 5 µg/mL. The diluted ligand-modified and mRNA-encapsulating LNP was intravenously administered to 4-week-old female Ai14 mouse (mentioned above) at a dose of 10 µL per 1 g of body weight (mRNA dose: 0.05 mg/kg). Three days after administration, the spleen was collected from the mouse and a cell suspension was produced by the method described in [Experimental Example 1]. 2×10⁶ cells were dispensed, and Fc blocking was performed. For Fc blocking, CD16/32 antibody (mentioned above) was added in the amount recommended by the manufacturer, and incubated on ice for 10 min or more. Brilliant Violet 605 anti-mouse CD45, Brilliant Violet 510 anti-mouse CD19, and Brilliant Violet 421 anti-mouse CD3ε (mentioned above) were added in the manufacturer's recommended amounts, and fluorescent labeling of cell surface antigens was performed by incubating on ice for 30 min. With 5 min remaining for antibody staining, 7-AAD Viability Staining Solution (mentioned above) was added in the manufacturer's recommended amount to perform dead cell staining. After washing the cells twice with FACS buffer, they were resuspended in FACS buffer (400 µL), subsets were classified using a Novocyte flow cytometer (ACEA Biosciences), and tdTomato expression in T cells (CD45+/CD3+/CD19-) was analyzed.

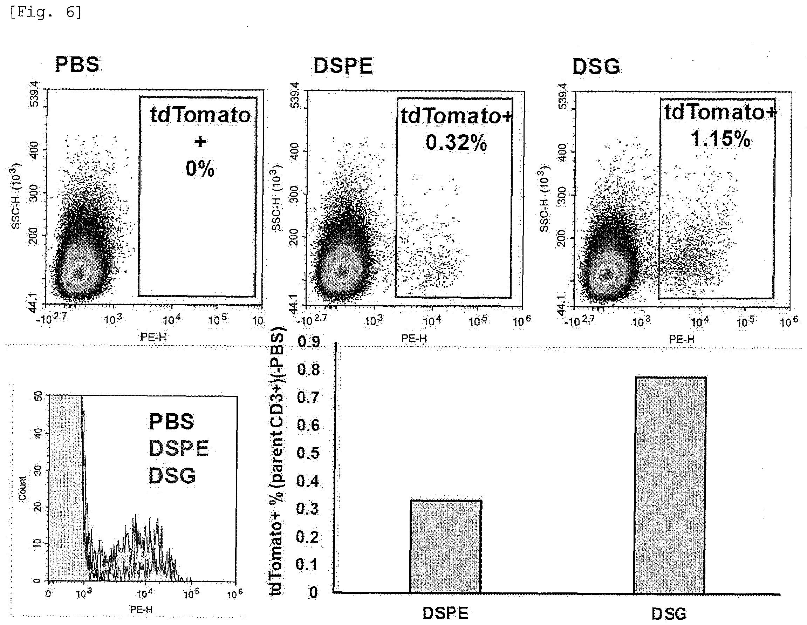

The results are shown in Fig. 6. In Fig. 6, "DSPE" or "DSG" indicates the results using DSPE-PEG2k-DBCO or DSG-PEG2k-DBCO, and "PBS" in Fig. 6 indicates the results not using DSPE-PEG2k-DBCO or DSG-PEG2k-DBCO. Uptake into T cells was better when DSPE-PEG2k-DBCO was used as activated PEG lipid than when DSG-PEG2k-DBCO was used. However, the number of cells expressing introduced mRNA was better when DSG-PEG2k-DBCO was used.

### [Reference Example 2] Fragmentation of CD19-IgG and introduction of reaction substrate

### 1. Fragmentation and purification of antibody

Anti-CD19 IgG antibody (CD19-IgG, source: Leinco) was desalted using a zeba-spin desalting column and replaced with sodium acetate buffer (20 mM, pH 4.5). The CD19-IgG after replacement was stirred at 37°C for 2 days at a ratio of CD19-IgG:pepsin=10 mg:0.125 mg to perform fragmentation. Separation from pepsin was performed by passing the fragment solution through an empty column. For the purpose of purification and buffer replacement, the fragment solution passed through the column was transferred to Amicon Ultra15 (MWCO: 50 kDa), diluted to 14 mL using sodium carbonate buffer (100 mM, pH 9.0), and centrifuged (25°C, 1000 g, 5 min) was repeated to ultrafiltrate and concentrate to about 500 µL. Thereafter, the volume was increased to 14 mL using sodium carbonate buffer (100 mM, pH 9.0), and centrifugation was repeated again (25°C, 1000 g, 5 min) to concentrate to 500 µL. Finally, the volume was increased to 1 mL using sodium carbonate buffer (100 mM, pH 9.0).

### 2. Introduction of reaction substrate

Protein concentration and protein amount were calculated by measuring the absorbance of the fragment solution at 280 nm using Nanodrop one (Thermo Scientific). 4 equivalents of Azido-PEG4-NHS were added to the fragment solution, and the mixture was shaken at room temperature for 2 hr and at 4°C overnight to introduce the reaction substrate. After introducing the reaction substrate, unreacted reagents were removed from the fragment solution using a zeba-spin desalting column and the solution was replaced with ultrapure water.

### 3. Confirmation of reaction substrate introduction

An amount equivalent to 100 µg of the fragment solution after introducing the substrate was taken into a light-shielding Eppendorf tube, 10 µL of ascorbic acid (20 mM), 10 µL of copper sulfate aqueous solution (20 mM), 2 µL of Cy5-Alkyne (10 mg/mL), and finally ultrapure water were added to increase the volume to 100 µL, and the mixture was reacted at room temperature for 2 hr. After the reaction, the reaction solution was transferred to a dialysis membrane with a cut-off molecular weight of 3.5 kDa, and dialysis was performed overnight. After dialysis, the amount of protein was calculated using the BCA method, and the amount of Cy5 modification was calculated based on fluorescence. The number of introduced reaction substrates was determined by dividing the amount of Cy5 modification by the amount of protein.

### [Example 4] Preparation of B-cell-directional ligand-modified and mRNA-encapsulating LNP

mRNA-encapsulating LNP (molar ratio of O-Ph-P4C2:DOPC:Chol = 52.5:7.5:40, and further using 1.5 mol% DMG-PEG2k, 0.4, 0.8, 1.2, or 1.6 mol% DSPE-PEG2k-DBCO (manufactured by Avanti Polar Lipids), and 0.5 mol% DiD as an index of uptake, each to the total of O-Ph-P4C2, DOPC, and Chol) was produced by the method described in [Example 1]. Thereafter, the LNP was modified by the method of [Example 1] using F(ab')2 produced from an anti-CD19-IgG antibody as a ligand to prepare ligand-modified and mRNA-encapsulating LNP.

### [Experimental Example 8] Evaluation of LNP uptake ability of splenic B cells in vivo

The ligand-modified and mRNA-encapsulating LNP prepared in Example 4 was diluted with PBS to an mRNA concentration of 1.5 µg/mL. The diluted ligand-modified and mRNA-encapsulating LNP was intravenously administered to 7-week-old female C57/BL6J mouse at a dose of 10 µL per 1 g of body weight (mRNA dose: 0.015 mg/kg). One day after administration, the spleen was collected from the mouse and a cell suspension was produced by the method described in [Experimental Example 1]. 1×10⁶ cells were subjected to Fc blocking by the method of [Experimental Example 1]. Brilliant Violet 605 anti-mouse CD45, Brilliant Violet 510 anti-mouse B220, and Brilliant Violet 421 anti-mouse CD3ε (mentioned above) were added in the manufacturer's recommended amounts, and fluorescent labeling of cell surface antigens was performed by incubating on ice for 30 min. With 5 min remaining for antibody staining, 7-AAD Viability Staining Solution (mentioned above) was added in the manufacturer's recommended amount to perform dead cell staining. After washing the cells twice with FACS buffer, they were resuspended in FACS buffer (400 µB), subsets were classified using a Novocyte flow cytometer (ACEA Biosciences), and uptake of LNP containing each concentration of activated PEG lipid into B cells (CD45+/CD3-/CD19+) was analyzed using DiD fluorescence as an index.

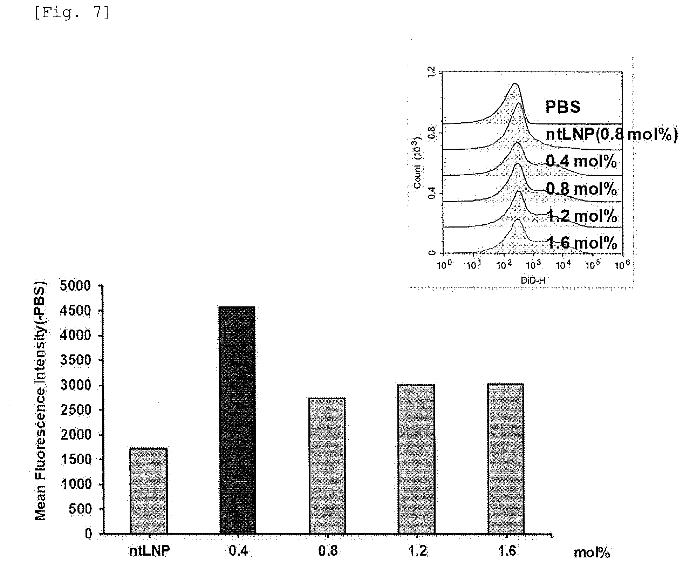

The results are shown in Fig. 7. In Fig. 7, "ntLNP" indicates the results using unmodified LNP, and "0.4(mol%)", "0.8 (mol%)", "1.2 (mol%)", or "1.6 (mol%)" in Fig. 7 each indicates the results using ligand-modified LNP in which the molar ratio of activated PEG lipid (DSPE-PEG2k-DBCO) to the total lipid amount is 0.4 mol%, 0.8 mol%, 1.2 mol%, or 1.6 mol%. When the molar ratio of activated PEG lipid (DSPE-PEG2k-DBCO) to the total lipid amount was 0.4 mol%, the uptake into B cells was highest, and the uptake into B cells significantly increased than that of unmodified LNP.

### [Experimental Example 9] Evaluation of LNP uptake ability of peripheral blood B cells in vivo

The ligand-modified and mRNA-encapsulating LNP prepared by the method described in Example 4 was diluted with PBS to an mRNA concentration of 1.5 µg/mL. The diluted ligand-modified and mRNA-encapsulating LNP was intravenously administered to 7-week-old female C57/BL6J mouse at a dose of 10 µL per 1 g of body weight (mRNA dose: 0.015 mg/kg). One day after administration, 20 µL of blood sample was collected from the mouse tail vein, a cell suspension was produced and Fc blocking was performed by the methods of [Experimental Example 1]. Brilliant Violet 605 anti-mouse CD45, Brilliant Violet 421 anti-mouse CD3ε (mentioned above) and Brilliant Violet 510 anti-mouse B220 (source: BioLegened) were added in the manufacturer's recommended amounts, and fluorescent labeling of cell surface antigens was performed by incubating on ice for 30 min. With 5 min remaining for antibody staining, 7-AAD Viability Staining Solution (mentioned above) was added in the manufacturer's recommended amount to perform dead cell staining. After washing the cells twice with FACS buffer, they were resuspended in FACS buffer (400 µL), subsets were classified using a Novocyte flow cytometer (ACEA Biosciences), and uptake into B cells (CD45+/CD3-/CD19+) was analyzed.

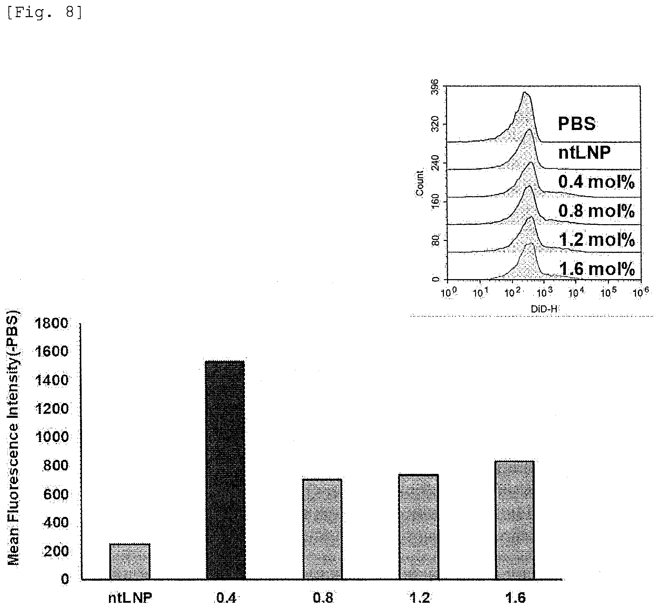

The results are shown in Fig. 8. In Fig. 8, "ntLNP" indicates the results using unmodified LNP, and "0.4 (mol%)", "0.8 (mol%)", "1.2 (mol%)", or "1.6 (mol%)" in Fig. 8 each indicates the results using ligand-modified LNP in which the molar ratio of activated PEG lipid (DSPE-PEG2k-DBCO) to the total lipid amount is 0.4 mol%, 0.8 mol%, 1.2 mol%, or 1.6 mol%. Similar to splenic B cells and also in peripheral blood B cells, when the molar ratio of activated PEG lipid (DSPE-PEG2k-DBCO) to the total lipid amount was 0.4 mol%, the uptake of LNP was highest, and the uptake into B cells significantly increased than that of unmodified LNP.

### [Experimental Example 10] Evaluation of LNP uptake ability in vivo

### 1. Preparation of LNP

Similar to Example 4, mRNA-encapsulating LNP (molar ratio of O-Ph-P4C2:DOPC:Chol = 52.5:7.5:40, and further using 1.5 mol% of DMG-PEG2k, 0.4, 0.8, 1.2, or 1.6 mol% of DSG-PEG2k-DBCO, and 0.5 mol% of DiD as an index of uptake, with respect to the total of O-Ph-P4C2, DOPC, and Chol) was produced by the method described in [Example 1]. Thereafter, the LNP was modified by the method of [Example 1] and using F(ab')2 produced from an anti-CD19-IgG antibody as a ligand to prepare ligand-modified and mRNA-encapsulating LNP.

### 2. Evaluation of LNP uptake ability of splenic B cells in vivo

The ligand-modified and mRNA-encapsulating LNP prepared in the above-mentioned 1. was diluted with PBS to an mRNA concentration of 1.5 µg/mL. The diluted ligand-modified and mRNA-encapsulating LNP was intravenously administered to 7-week-old female C57/BL6J mouse at a dose of 10 µL per 1 g of body weight (mRNA dose: 0.015 mg/kg). One day after administration, the spleen was collected from the mouse and a cell suspension was produced by the method described in [Experimental Example 8]. 1×10⁶ cells were subjected to Fc blocking and antibody staining by the method of [Experimental Example 8]. Subsets were classified using a Novocyte flow cytometer (ACEA Biosciences), and uptake of LNP containing each concentration of activated PEG lipid into splenic B cells (CD45+/CD3-/CD19+) was analyzed.

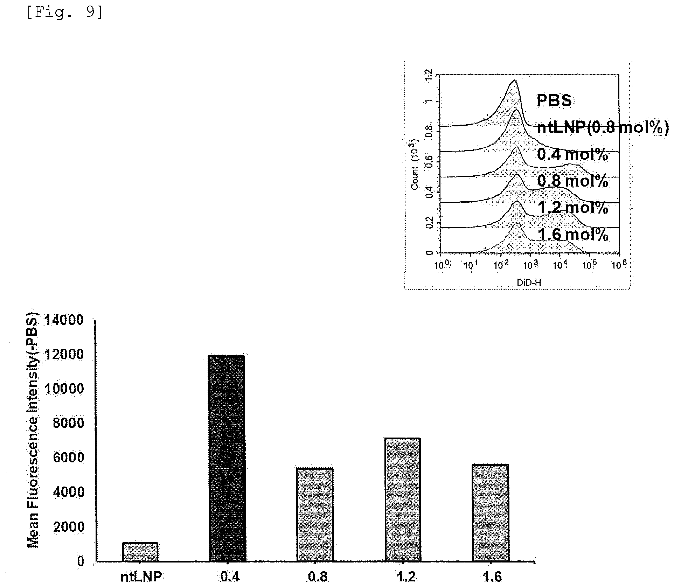

The results are shown in Fig. 9. In Fig. 9, ntLNP indicates the results using unmodified LNP, and "0.4 (mol%)", "0.8 (mol%)", "1.2 (mol%)", or "1.6 (mol%)" in Fig. 9 each indicates the results using ligand-modified LNP in which the molar ratio of activated PEG lipid (DSG-PEG2k-DBCO) to the total lipid amount is 0.4 mol%, 0.8 mol%, 1.2 mol%, or 1.6 mol%. At all investigated molar ratios of activated PEG lipids (DSG-PEG2k-DBCO) to total lipids (0.4 to 1.6 mol%), ligand-modified LNPs significantly increased the uptake into B cells compared to unmodified LNP. In particular, when the molar ratio was 0.4 mol%, the uptake into B cells was highest, and the uptake into B cells also increased significantly for other molar ratios (0.8 to 1.6%).

### [Experimental Example 11] Evaluation of LNP uptake ability of peripheral blood B cells in vivo

The ligand-modified and mRNA-encapsulating LNP prepared by the method described in Experimental Example 10, 1., was diluted with PBS to an mRNA concentration of 1.5 µg/mL. The diluted ligand-modified and mRNA-encapsulating LNP was intravenously administered to 7-week-old female C57/BL6J mouse at a dose of 10 µL per 1 g of body weight (mRNA dose: 0.015 mg/kg). One day after administration, 20 µL of blood sample was collected from the mouse tail vein, a cell suspension was produced by the methods of [Experimental Example 9]. Antibody staining was performed by the method of [Experimental Example 9]. Subsets were classified using a Novocyte flow cytometer (ACEA Biosciences), and uptake of LNP containing each concentration of activated PEG lipid into peripheral blood B cells (CD45+/CD3-/CD19+) was analyzed.

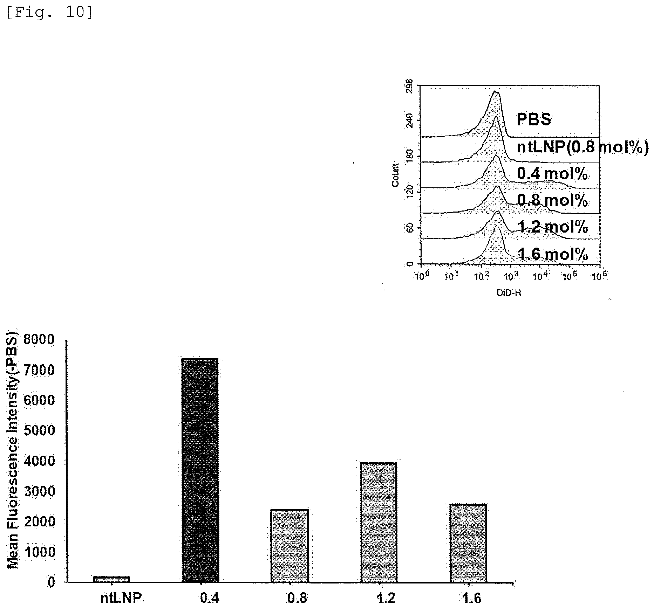

The results are shown in Fig. 10. In Fig. 10, ntLNP indicates the results using unmodified LNP, and "0.4 (mol%)", "0.8 (mol%)", "1.2 (mol%)", or "1.6 (mol%)" in Fig. 10 each indicates the results using ligand-modified LNP in which the molar ratio of activated PEG lipid (DSG-PEG2k-DBCO) to the total lipid amount is 0.4 mol%, 0.8 mol%, 1.2 mol%, or 1.6 mol%. At all investigated molar ratios of activated PEG lipids (DSG-PEG2k-DBCO) to total lipids (0.4 to 1.6 mol%), ligand-modified LNPs significantly increased the uptake into B cells compared to unmodified LNP. In particular, when the molar ratio was 0.4 mol%, the uptake into B cells was highest, and the uptake into B cells also increased significantly for other molar ratios (0.8 to 1.6%).

### [Industrial Applicability]

The lipid nanoparticles of the present invention are useful for nucleic acid medicament, gene therapy, biochemical experiment, and the like.

This application is based on a patent application No. 2021-161235 filed in Japan, the contents of which are incorporated in full herein.

## Claims

1. A lipid nanoparticle comprising
an ionic lipid represented by the formula (1): 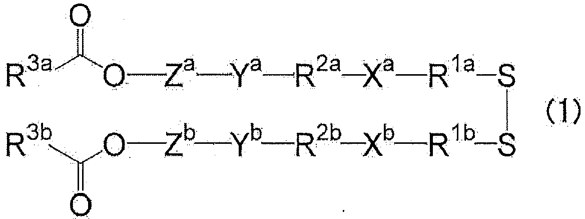 (in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom, and
R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms), and
a ligand having cell directivity and bonded to an activated PEG lipid.

2. The lipid nanoparticle according to claim 1, wherein the activated PEG lipid is represented by the formula (2): 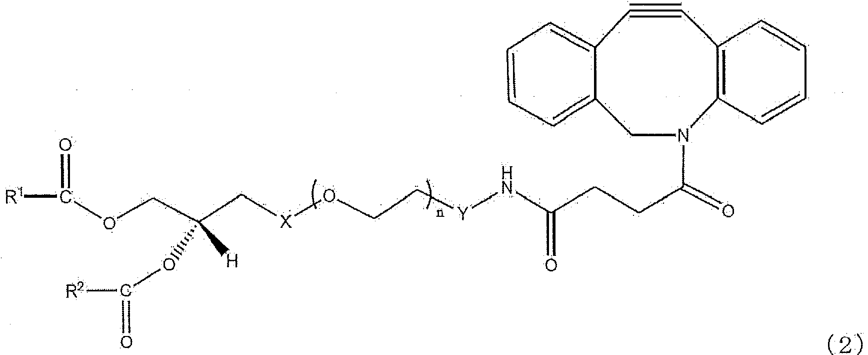 (in the formula (2),
R¹ and R² are each independently a hydrocarbon chain having 14 to 18 carbon atoms,
X is a group represented by the formula (3): 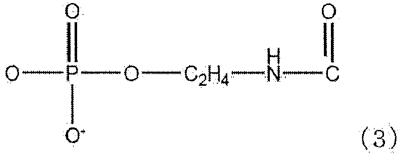 or a single bond,
when X is a group represented by the formula (3), then Y is a single bond,
when X is a single bond, then Y is an oxygen atom, and
n is an integer that satisfies the molecular weight of (OCH₂CH₂)ₙ of 1000 to 5000.)

3. The lipid nanoparticle according to claim 2, wherein in the formula (2), R¹ and R² are each a saturated hydrocarbon chain having 18 carbon atoms, X is a single bond, and Y is an oxygen atom.

4. The lipid nanoparticle according to any one of claims 1 to 3, wherein the ligand is a peptide or antibody that has specific affinity for a molecule expressed on the surface of the target cell.

5. The lipid nanoparticle according to claim 4, wherein the ligand is an anti-CD3 antibody.

6. The lipid nanoparticle according to claim 4, wherein the ligand is an anti-CD19 antibody.

7. The lipid nanoparticle according to any one of claims 1 to 5, wherein the ligand has directivity to T cells.

8. The lipid nanoparticle according to any one of claims 1 to 4 and 6, wherein the ligand has directivity to B cells.

9. A method for producing a lipid nanoparticle modified with a ligand having cell directivity, comprising
producing a lipid nanoparticle from a lipid material comprising an ionic lipid represented by the formula (1): 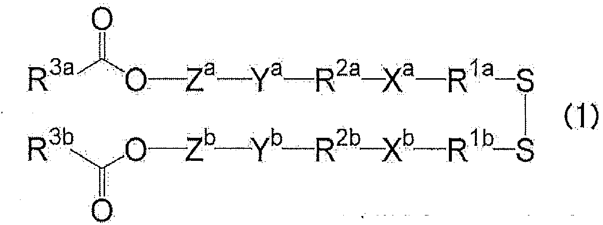 (in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom, and
R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms),
and an activated PEG lipid, and
binding the ligand to the obtained lipid nanoparticle.

10. The method according to claim 9, wherein the activated PEG lipid is represented by the formula (2): 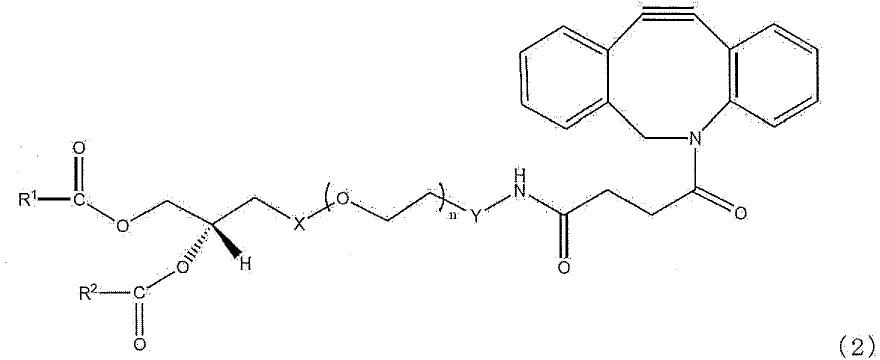 (in the formula (2),
R¹ and R² are each independently a hydrocarbon chain having 14 to 18 carbon atoms,
X is a group represented by the formula (3): 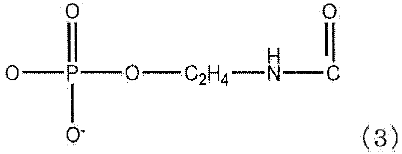 or a single bond,
when X is the group represented by the formula (3), then Y is a single bond,
when X is a single bond, then Y is an oxygen atom, and
n is an integer that satisfies the molecular weight of (OCH₂CH₂)ₙ of 1000 to 5000).

11. The method according to claim 10, wherein in the formula (2), R¹ and R² are each a saturated hydrocarbon chain having 18 carbon atoms, X is a single bond, and Y is an oxygen atom.

12. A nucleic acid-introducing agent comprising the lipid nanoparticles according to any one of claims 1 to 8.

13. A method for introducing a nucleic acid into a cell, comprising contacting the nucleic acid-introducing agent according to claim 12 encapsulating the nucleic acid with the cell in vitro.

14. The method according to claim 13, wherein the cell is a T cell or a B cell.

15. A method for introducing a nucleic acid into a cell, comprising administering the nucleic acid-introducing agent according to claim 12 encapsulating the nucleic acid to a living body so that the agent is delivered to the target cell.

16. The method according to claim 15, wherein the target cell is a T cell or a B cell.
